(19) Europäisches Patentamt
European Patent Office
Office européen des brevets

(11) EP 3 011 897 A1

(12) EUROPEAN PATENT APPLICATION

(43) Date of publication:
27.04.2016 Bulletin 2016/17

(51) Int Cl.:
A61B 5/00 (2006.01)    A61B 8/08 (2006.01)

(21) Application number: 15186402.2

(22) Date of filing: 23.09.2015

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 21.10.2014 JP 2014214221

(71) Applicant: PreXion Corporation
Tokyo 101-0041 (JP)

(72) Inventor: AGANO, Toshitaka
Chiyoda-ku, Tokyo 101-0041 (JP)

(74) Representative: Grünecker Patent- und
Rechtsanwälte
PartG mbB
Leopoldstraße 4
80802 München (DE)

(54) PHOTOACOUSTIC IMAGER AND PHOTOACOUSTIC IMAGING METHOD

(57) A photoacoustic imager (100, 200, 300, 400) includes a light source portion (11), a detection portion (12, 26, 226), and an imaging portion (25, 225, 325, 425), and the imaging portion is configured to generate a photoacoustic wave image indicating a detection object in motion by acquiring difference data of signals acquired on the basis of a plurality of photoacoustic wave signals detected at different times of generated photoacoustic wave signals.

FIG.4 FIRST EMBODIMENT (MODIFICATION)

EP 3 011 897 A1

**Description**

BACKGROUND OF THE INVENTION

Field of the Invention

The present invention relates to a photoacoustic imager and a photoacoustic imaging method, and more particularly, it relates to a photoacoustic imager including a detection portion that detects an acoustic wave generated by light applied to a specimen and a photoacoustic imaging method.

Description of the Background Art

A photoacoustic imager including a detection portion that detects an acoustic wave generated by light applied to a specimen is known in general, as disclosed in Japanese Patent Laying-Open No. 2013-075000, for example.

The aforementioned Japanese Patent Laying-Open No. 2013-075000 discloses a photoacoustic image generator including an ultrasonic probe that detects a photoacoustic wave signal resulting from a laser beam applied to a specimen. This photoacoustic image generator is provided with a laser unit and an ultrasonic unit. The photoacoustic image generator is configured to apply a laser beam from the laser unit to the specimen and to detect a photoacoustic wave signal generated from a detection object in the specimen by the ultrasonic probe. The ultrasonic unit includes a photoacoustic image generation means, and the photoacoustic image generation means is configured to generate a photoacoustic wave image on the basis of the photoacoustic wave signal detected by the ultrasonic probe. Thus, the photoacoustic image generator is configured to be capable of generating a photoacoustic wave image indicating whether or not the detection object exists in the specimen on the basis of the photoacoustic wave signal.

Although the photoacoustic image generator according to the aforementioned Japanese Patent Laying-Open No. 2013-075000 can generate the photoacoustic wave image indicating whether or not the detection object exists in the specimen on the basis of the photoacoustic wave signal, the photoacoustic image generator cannot generate a photoacoustic wave image indicating the detection object in motion in the specimen on the basis of the photoacoustic wave signal.

SUMMARY OF THE INVENTION

The present invention has been proposed in order to solve the aforementioned problem, and an object of the present invention is to provide a photoacoustic image generator capable of generating a photoacoustic wave image indicating a detection object in motion in a specimen on the basis of a photoacoustic wave signal.

In order to attain the aforementioned object, a photoacoustic imager according to a first aspect of the present invention includes a light source portion that applies light to a specimen, a detection portion that detects an acoustic wave generated by absorption of the light applied from the light source portion to the specimen by a detection object in the specimen and generates photoacoustic wave signals, and an imaging portion that generates a photoacoustic wave image indicating the detection object in motion by acquiring difference data of signals acquired on the basis of a plurality of photoacoustic wave signals detected at different times of the photoacoustic wave signals to extract portions in which the intensities of the photoacoustic wave signals temporally change.

As hereinabove described, the photoacoustic imager according to the first aspect of the present invention is configured to acquire the difference data of signals acquired on the basis of the plurality of photoacoustic wave signals detected at the different times of the photoacoustic wave signals, whereby data of an unmoving portion of the detection object is subtracted while data of a moving portion of the detection object remains. Therefore, the portions in which the intensities of the photoacoustic wave signals temporally change can be extracted. Thus, the photoacoustic wave image indicating the detection object in motion in the specimen can be generated on the basis of the photoacoustic wave signals.

In the aforementioned photoacoustic imager according to the first aspect, the imaging portion is preferably configured to acquire the photoacoustic wave signals at first time intervals, to acquire the difference data by calculating differences between signals based on the photoacoustic wave signals that are acquired and signals based on the photoacoustic wave signals that have been acquired immediately prior to the photoacoustic wave signals that are acquired, and to generate the photoacoustic wave image on the basis of the difference data that is acquired. According to this structure, the photoacoustic wave signals are acquired at the first time intervals, and hence the photoacoustic wave image of the detection object in the specimen moved during a prescribed time (first time) can be continuously repetitively generated. Difference calculation generally indicates calculation of a value of a difference between two signal values, but according to the present invention, difference calculation indicates a wide concept including not only calculation of a difference between two signal values but also calculation of a value obtained on the basis of a ratio of signal values.

In this case, the imaging portion is preferably configured to generate an averaged signal by averaging the photoacoustic wave signals acquired at the first time intervals, and is preferably configured to acquire the difference data by calculating a difference between a current averaged signal and an immediately prior averaged signal and to generate the photoacoustic wave image on the basis of the difference data that is acquired. According to this structure, the difference data can be acquired in a state where the signal-noise ratio of the photoacoustic wave signals is improved by averaging.

In the aforementioned photoacoustic imager that acquires the difference data by calculating the difference between the current averaged signal and the immediately prior averaged signal, the imaging portion is preferably configured such that a second time interval equal to or greater than each of the first time intervals is provided between a time point when the immediately prior averaged signal is generated and a time point when the current averaged signal is generated. According to this structure, the second time interval is provided, and hence the difference between the immediately prior averaged signal and the current averaged signal can be increased. Therefore, the difference data and the photoacoustic wave image more clearly indicating the detection object in motion can be generated.

In the aforementioned photoacoustic imager that acquires the photoacoustic wave signals at the first time intervals, each of the first time intervals is preferably at least 0.1 msec and not more than 100 msec. The blood flow velocity of blood (detection object) in a human body (specimen) is generally at least 1 mm/s and not more than 1000 mm/s. The resolution of imaging of a common photoacoustic imager is within a range from several 10 $\mu$m order to several mm order. In consideration of this point, as in the present invention, when the first time intervals are set to at least 0.1 msec, the moving distance of the aforementioned blood is at least 0.1 $\mu$m and not more than 100 $\mu$m. Thus, the blood having a relatively large blood flow velocity (blood flow velocity of 1000 mm/s, for example) can be observed in correspondence to the resolution of imaging of the common photoacoustic imager. Furthermore, as in the present invention, when the first time intervals are set to not more than 100 msec, the moving distance of the aforementioned blood is at least 100 $\mu$m and not more than 100 mm. Thus, the blood having a relatively small blood flow velocity (blood flow velocity of 1 mm/s, for example) can be observed in correspondence to the resolution of imaging of the common photoacoustic imager. Therefore, the first time intervals are set to at least 0.1 msec and not more than 100 msec, whereby the photoacoustic wave image indicating the movement of the blood in the human body can be properly generated in correspondence to the resolution of imaging of the photoacoustic imager.

In this case, each of the first time intervals is preferably at least 1 msec and not more than 50 msec. According to this structure, the moving distance of the aforementioned blood is within a range from 1 $\mu$m to 1 mm when the first time intervals are set to 1 msec, and the moving distance of the aforementioned blood is within a range from 50 $\mu$m to 50 mm when the first time intervals are set to 50 msec, whereby the photoacoustic wave image can be generated in closer correspondence to the resolution of imaging of the photoacoustic imager.

In the aforementioned photoacoustic imager according to the first aspect, the detection portion is preferably configured to generate the photoacoustic wave signals including RF signals on the basis of the acoustic wave that is detected, and the imaging portion is preferably configured to generate the photoacoustic wave image on the basis of the difference data acquired on the basis of a plurality of RF signals detected at different times of the RF signals. Generally, fine information (such as information indicating the phases of signals) contained in the RF (radio frequency) signals may be lost when the RF signals are demodulated (detected). On the other hand, as in the present invention, when the photoacoustic wave image is generated on the basis of the difference data acquired on the basis of the plurality of RF signals detected at the different times of the RF signals, the photoacoustic wave image can be generated without losing the fine information contained in the RF signals. Consequently, the photoacoustic wave image faithfully indicating the movement of the detection object can be generated. The RF signals generally denote high-frequency signals, but in this description, the RF signals denote high-frequency signals that are non-demodulated (non-detected) RF signals.

In the aforementioned photoacoustic imager according to the first aspect, the detection portion is preferably configured to generate RF signals on the basis of the acoustic wave that is detected and to generate the photoacoustic wave signals including demodulation signals obtained by demodulating the RF signals, and the imaging portion is preferably configured to generate the photoacoustic wave image on the basis of the difference data acquired on the basis of a plurality of demodulation signals detected at different times of the demodulation signals. According to this structure, the data capacity of the demodulation signals is smaller than that of the RF signals, and hence the capacity of the difference data can be reduced. Consequently, an increase in the capacity of memories of the imaging portion for storing the difference data can be significantly reduced or prevented.

The aforementioned photoacoustic imager according to the first aspect preferably further includes a display portion that displays the photoacoustic wave image, and the imaging portion is preferably configured to acquire the photoacoustic wave signals at first time intervals, to set a plurality of third time intervals that are equal to or greater than the first time intervals and are different from each other, to generate photoacoustic wave images corresponding to the plurality of respective third time intervals, to select the photoacoustic wave image having the highest image definition from the photoacoustic wave images that are generated, and to output the photoacoustic wave image that is selected to the display portion. According to this structure, a user can visually recognize the photoacoustic wave image with the highest image definition even when a time interval in which the image definition becomes highest is varied according to the movement (such as the velocity) of the detection object.

In the aforementioned photoacoustic imager according to the first aspect, the imaging portion is preferably configured to generate a plurality of photoacoustic wave images, to perform non-linear processing for performing at least one of processing for reducing a noise component contained in each of the plurality of photoacoustic wave images and processing for enhancing a signal component contained in each of the plurality of photoacoustic wave images, and to synthesize the plurality of photoacoustic wave images that are non-linearly processed. According to this structure, the photoacoustic wave image can be generated while the signal component with respect to the noise component is increased in the photoacoustic wave image by the non-linear processing. Furthermore, the plurality of non-linearly processed photoacoustic wave images are synthesized, whereby the photoacoustic wave image in which the locus of the movement of the detection object is further emphasized can be generated.

In this case, the imaging portion is preferably configured to perform the non-linear processing that is the processing for reducing the noise component contained in each of the photoacoustic wave images and processing for enhancing the signal component contained in each of the photoacoustic wave images by multiplying a value of each piece of data of the photoacoustic wave image by a correction coefficient Z expressed by a following formula (1), $Z = a(W) + 1 \ldots (1)$, setting a function expressing the amplitude W of a photoacoustic wave signal as a variable as $\underline{a}$. When the amplitude W of the photoacoustic wave signal is small, the photoacoustic wave signal often becomes the noise component in the photoacoustic wave image, and when the amplitude W of the photoacoustic wave signal is large, the photoacoustic wave signal often becomes the signal component in the photoacoustic wave image. Focusing on this point, according to the present invention, by multiplying the value of each piece of data of the photoacoustic wave image by the correction coefficient Z expressed by the aforementioned formula (1), the noise component contained in the photoacoustic wave image can be effectively reduced while the signal component contained in the photoacoustic wave image can be effectively enhanced.

The aforementioned photoacoustic imager according to the first aspect preferably further includes a display portion that displays the photoacoustic wave image, and the imaging portion is preferably configured to output the photoacoustic wave image generated on the basis of the difference data and not synthesized to the display portion at a fourth time interval. According to this structure, no processing for synthesizing the photoacoustic wave images is performed, and hence a processing load on the imaging portion can be reduced.

The aforementioned photoacoustic imager according to the first aspect preferably further includes a display portion that displays the photoacoustic wave image, and the detection portion is preferably configured to generate an ultrasonic wave to be applied to the specimen, to detect the ultrasonic wave applied to the specimen and reflected in the specimen, and to generate an ultrasonic detection signal, and the imaging portion is preferably configured to superpose a first photoacoustic wave image generated on the basis of the difference data and at least one of a second photoacoustic wave image acquired by imaging a photoacoustic wave signal and an ultrasonic image acquired by imaging the ultrasonic detection signal and to output a superposed image to the display portion. According to this structure, at least one of the second photoacoustic wave image and the ultrasonic image that are images indicating whether or not the detection object exists in the specimen and the first photoacoustic wave image that is an image indicating the detection object in motion are superposed to be displayed on the display portion, and hence the user can visually recognize the position of the detection object in the specimen and the movement of the detection object associated with each other.

In the aforementioned photoacoustic imager according to the first aspect, the light source portion preferably includes any of a light-emitting diode element, a semiconductor laser element, and an organic light-emitting diode element. According to this structure, the light-emitting diode element, the semiconductor laser element, and the organic light-emitting diode element can apply light whose repetition frequency is relatively high (at least 1 kHz, for example), unlike a solid-state laser light source that applies pulsed light whose repetition frequency is about 10 Hz. Consequently, a time interval in which light is applied can be reduced, and hence the photoacoustic wave image indicating the detection object that is traveling a long distance in a relatively short amount of time (whose moving velocity is large) can be also generated.

A photoacoustic imaging method according to a second aspect of the present invention includes steps of applying light from a light source portion to a specimen, detecting an acoustic wave generated by absorption of the light applied from the light source portion to the specimen by a detection object in the specimen and generating photoacoustic wave signals, and generating a photoacoustic wave image indicating the detection object in motion by acquiring difference data of signals acquired on the basis of a plurality of photoacoustic wave signals detected at different times of the photoacoustic wave signals to extract portions in which intensities of the photoacoustic wave signals temporally change.

In the photoacoustic imaging method according to the second aspect of the present invention, as hereinabove described, the difference data of signals acquired on the basis of the plurality of photoacoustic wave signals detected at the different times of the photoacoustic wave signals is acquired, whereby the portions in which the intensities of the photoacoustic wave signals temporally change are extracted. Thus, the photoacoustic wave image indicating the detection object in motion in the specimen can be generated on the basis of the photoacoustic wave signals also by the photoacoustic imaging method according to the second aspect.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a block diagram showing the overall structure of a photoacoustic imager according to a first embodiment of the present invention;

Fig. 2 illustrates acquisition of detection signals by an ultrasonic vibrator portion according to the first embodiment of the present invention;

Fig. 3 illustrates generation of photoacoustic wave signals by a receiving circuit according to the first embodiment of the present invention;

Fig. 4 is a block diagram of a portion of the photoacoustic imager according to the first embodiment of the present invention, involved in generation of a photoacoustic wave image;

Fig. 5 illustrates generation of difference data by an imaging portion according to the first embodiment of the present invention;

Fig. 6 illustrates generation and reconstruction of the difference data by the imaging portion according to the first embodiment of the present invention;

Fig. 7 is a diagram for illustrating non-linear processing performed by the imaging portion according to the first embodiment of the present invention;

Fig. 8 is another diagram for illustrating the non-linear processing performed by the imaging portion according to the first embodiment of the present invention;

Fig. 9 illustrates a plurality of time intervals according to the first embodiment of the present invention;

Fig. 10 illustrates image analysis processing performed by the imaging portion according to the first embodiment of the present invention;

Fig. 11 is a diagram for illustrating a display image displayed on an image display portion according to the first embodiment of the present invention;

Fig. 12 is a diagram for illustrating another display image displayed on the image display portion according to the first embodiment of the present invention;

Fig. 13 is a flowchart for illustrating imaging processing for the photoacoustic wave image according to the first embodiment of the present invention;

Fig. 14 is a block diagram of a portion of a photoacoustic imager according to a second embodiment of the present invention, involved in generation of a photoacoustic wave image;

Fig. 15 is a block diagram of a portion of a photoacoustic imager according to a third embodiment of the present invention, involved in generation of a photoacoustic wave image;

Fig. 16 is a block diagram of a portion of a photoacoustic imager according to a fourth embodiment of the present invention, involved in generation of a photoacoustic wave image; and

Fig. 17 is a diagram for illustrating non-linear processing performed by an imaging portion according to a modification of the first embodiment of the present invention.

DESCRIPTION OF THE PREFERRED EMBODIMENTS

Embodiments of the present invention are hereinafter described with reference to the drawings.

(First Embodiment)

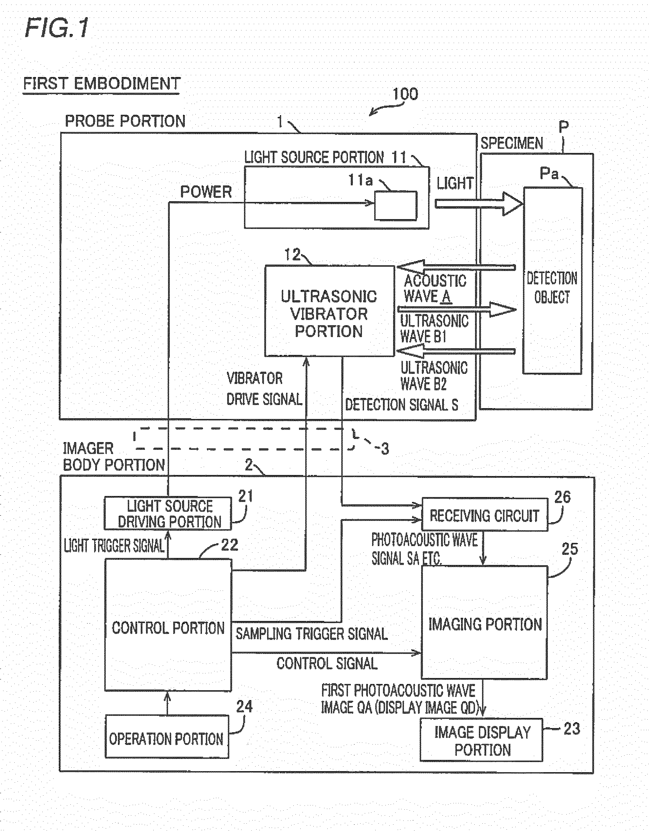

The overall structure of a photoacoustic imager 100 according to a first embodiment of the present invention is now described with reference to Figs. 1 to 12. According to the first embodiment, the photoacoustic imager 100 has a function of generating a first photoacoustic wave image QA indicating a detection object Pa (such as blood) in motion in a specimen P (such as a human body).

The photoacoustic imager 100 according to the first embodiment of the present invention is provided with a probe portion 1 and an imager body portion 2, as shown in Fig. 1. The photoacoustic imager 100 is also provided with a cable 3 connecting the probe portion 1 and the imager body portion 2 to each other.

The probe portion 1 is so configured that the same is grasped by an operator and arranged on a surface of the specimen P (such as a surface of the human body). Furthermore, the probe portion 1 is configured to be capable of applying light to the specimen P, to detect an acoustic wave $\underline{A}$ and an ultrasonic wave B2, both described later, from the detection object Pa in the specimen P, and to transmit the acoustic wave A and the ultrasonic wave B2 as detection signals S to the imager body portion 2 through the cable 3.

The imager body portion 2 is configured to process and image the detection signals S (a photoacoustic wave signal SA and an ultrasonic signal SB both described later) detected by the probe portion 1 and to display the imaged acoustic wave A (the first photoacoustic wave image QA and a second photoacoustic wave image QC both described later) and ultrasonic wave B2 (an ultrasonic image QB).

According to the first embodiment, the photoacoustic imager 100 is configured to generate the first photoacoustic wave image QA indicating the detection object Pa in motion by acquiring difference data D of signals acquired on the basis of a plurality of photoacoustic wave signals SA detected at different times of photoacoustic wave signals SA to extract portions in which the intensities of the photoacoustic wave signals SA (acoustic waves A) temporally change.

The structure of the photoacoustic imager 100 is now described in detail.

The probe portion 1 is provided with a light source portion 11. According to the first embodiment, the light source portion 11 includes a plurality of semiconductor light-emitting elements 11a. The semiconductor light-emitting elements 11a include any of light-emitting diode elements, semiconductor laser elements, and organic light-emitting diode elements. The semiconductor light-emitting elements 11a are configured to be capable of emitting pulsed light having a wavelength (a wavelength of about 850 nm, for example) in the infrared region by being supplied with power from a light source driving portion 21 described later. The light source portion 11 is configured to apply the light emitted from the plurality of semiconductor light-emitting elements 11a to the specimen P.

The imager body portion 2 is provided with the light source driving portion 21. The light source driving portion 21 is configured to acquire power from an external power source (not shown). The light source driving portion 21 is further configured to supply power to the light source portion 11 on the basis of a light trigger signal received from a control portion 22 described later. The light trigger signal is configured as a signal whose frequency is 1 kHz, for example. Thus, the light source portion 11 is configured to apply pulsed light whose repetition frequency is 1 kHz to the specimen P. The light source driving portion 21 is configured to be capable of supplying power whose frequency is at least 1 kHz to the light source portion 11 even when acquiring a light trigger signal whose frequency is at least 1 kHz.

The imager body portion 2 is also provided with the control portion 22, an image display portion 23, and an operation portion 24. The control portion 22 is configured to control operations of each portion of the photoacoustic imager 100. The image display portion 23 is configured to be capable of displaying the first photoacoustic wave image QA, the second photoacoustic wave image QC, and the ultrasonic image QB each generated by an imaging portion 25 described later. The operation portion 24 is configured to accept input operations on the photoacoustic imager 100 from the operator. The control portion 22 is configured to perform processing for switching the type of an image displayed on the image display portion 23 as described later on the basis of information about the input operations by the operator accepted through the operation portion 24, for example.

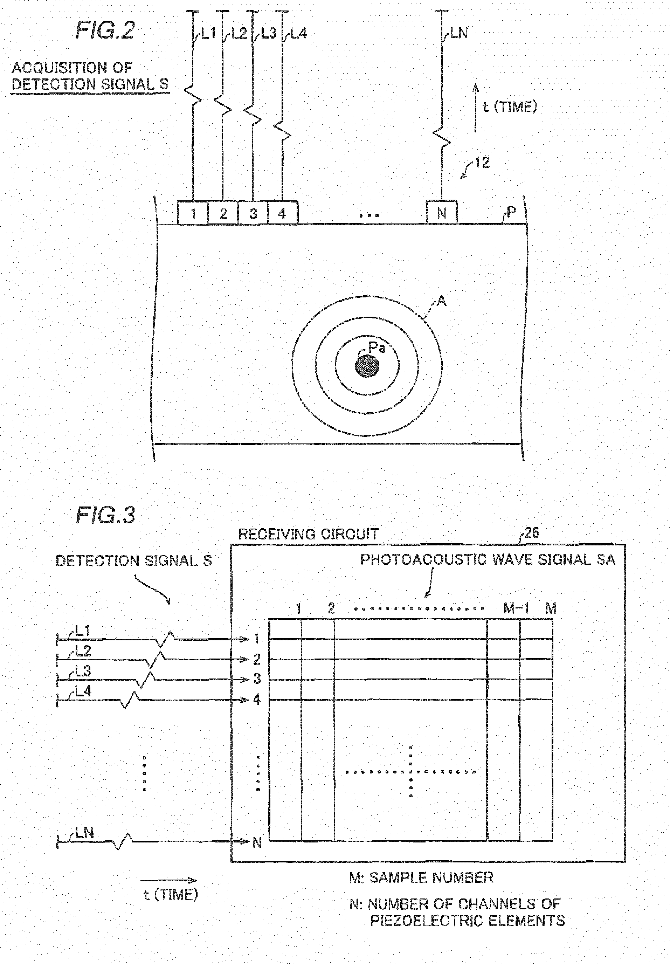

The probe portion 1 is also provided with an ultrasonic vibrator portion 12. As shown in Fig. 2, the ultrasonic vibrator portion 12 includes piezoelectric elements (lead zirconate titanate (PZT), for example) of N channels (N piezoelectric elements). The number N of channels in the ultrasonic vibrator portion 12 is 64, 128, 192, or 256, for example. Element intervals in the ultrasonic vibrator portion 12 are within a range from several 10 $\mu$m to 1 mm. Thus, the resolution of imaging of the photoacoustic imager 100 is within a range from several 10 $\mu$m order to several mm order, for example. The ultrasonic vibrator portion 12 is an example of the "detection portion" in the present invention.

The detection object Pa (such as hemoglobin in the blood) in the specimen P absorbs the pulsed light applied from the probe portion 1 to the specimen P. The detection object Pa generates the acoustic wave A by expanding and contracting (returning to the original size from an expanding state) in response to the intensity of application (the quantity of absorption) of the pulsed light.

According to the first embodiment, the ultrasonic vibrator portion 12 is configured to detect the acoustic wave A generated by the absorption of the light applied from the light source portion 11 to the specimen P by the detection object Pa in the specimen P and to acquire a detection signal S.

Specifically, the piezoelectric elements of N channels in the ultrasonic vibrator portion 12 are configured to vibrate and acquire the detection signal S (RF (radio frequency) signal) when acquiring the acoustic wave A. Therefore, the detection signal S (RF signal) contains information about the channels of the piezoelectric elements and information about the signal intensity, the signal frequency, and the detection time t. The information about the channels of the piezoelectric elements corresponds to the positional information of the ultrasonic vibrator portion 12 in a width direction, and the detection time t corresponds to the positional information of the detection object Pa in a depth direction. The ultrasonic vibrator portion 12 is configured to transmit the acquired detection signal S (RF signal) to a receiving circuit 26 through each of signal lines L1 to LN by each of the channels.

According to the first embodiment, the ultrasonic vibrator portion 12 is configured to generate an ultrasonic wave B1 to be applied to the specimen P, to detect the ultrasonic wave B2 applied to the specimen P and reflected in the specimen P, and to generate a detection signal S, as shown in Fig. 1.

The ultrasonic vibrator portion 12 is configured to generate the ultrasonic wave B1 by vibrating at a frequency according to a vibrator drive signal from the control portion 22. The ultrasonic wave B1 generated by the ultrasonic vibrator portion 12 is reflected by a substance having a high acoustic impedance in the specimen P. The ultrasonic vibrator portion 12 is configured to detect the ultrasonic wave B2 (reflected ultrasonic wave B1) and to vibrate due to the ultrasonic wave B2. The ultrasonic vibrator portion 12 is configured to transmit the detection signal S to the receiving circuit 26 also when vibrating due to the ultrasonic wave B2, similarly to the case of vibrating due to the acoustic wave A. In this description, an ultrasonic wave generated by light absorption by the detection object Pa in the specimen P is referred to as the "acoustic wave A", and an ultrasonic wave generated by the ultrasonic vibrator portion 12 and reflected in the specimen P is distinctively referred to as the "ultrasonic wave B2" for the convenience of illustration.

The imager body portion 2 is provided with the receiving circuit 26. The receiving circuit 26 is connected to the ultrasonic vibrator portion 12 through the cable 3. According to the first embodiment, the receiving circuit 26 is configured to generate the photoacoustic wave signal SA including the RF signal on the basis of the detection signal S from the ultrasonic vibrator portion 12, as shown in Fig. 3. The receiving circuit 26 is an example of the "detection portion" in the present invention.

Specifically, the receiving circuit 26 includes a coupling capacitor, an A-D converter, etc. The coupling capacitor of the receiving circuit 26 is configured to acquire alternating-current components of the detection signals S from the ultrasonic vibrator portion 12. The A-D converter of the receiving circuit 26 is configured to convert the detection signals (analog signals) into digital signals. The receiving circuit 26 is configured to generate the photoacoustic wave signal SA and the ultrasonic signal SB from the detection signals S according to a sampling trigger signal (the sample number is M, for example) from the control portion 22.

For example, the photoacoustic wave signal SA includes data obtained by configuring information about the width direction of the ultrasonic vibrator portion 12 and information about the depth direction from the surface of the specimen P in a matrix, as shown in Fig. 3. Specifically, the photoacoustic wave signal SA is configured by a matrix of the N channels of the piezoelectric elements of the ultrasonic vibrator portion 12 and the sample number M. The sample number M corresponds to a depth desired to be imaged. When the depth desired to be imaged is 60 mm (0.06 m) from the surface of the specimen P, the sound velocity in the human body is 1500 m/s, and the sampling frequency of the sampling trigger signal is $20 \times 10^6$ Hz, for example, the sample number M is 800 (= $(0.06/1500) \times 20 \times 10^6$). The sample number M indicates the number of pixels in the depth direction. In the case of the aforementioned calculation example, there are 800 pixels in the depth direction. One photoacoustic wave signal SA is generated per application of the pulsed light by the light source portion 11. The ultrasonic signal SB is also generated similarly to the photoacoustic wave signal SA, and one ultrasonic signal SB is generated per application of the ultrasonic wave B1. The photoacoustic wave signal SA and the ultrasonic signal SB are so-called projection signals.

The receiving circuit 26 is configured to transmit the photoacoustic wave signal SA and the ultrasonic signal SB to the imaging portion 25. The photoacoustic imager 100 is configured not to superpose a period for applying the pulsed light to the specimen P by the light source portion 11 so that the specimen P generates an acoustic wave A1 and the ultrasonic vibrator portion 12 acquires the acoustic wave A1 and a period for applying the ultrasonic wave B1 to the specimen P by the ultrasonic vibrator portion 12 so that the ultrasonic vibrator portion 12 acquires the ultrasonic wave B2, to be capable of distinguishing the detection signal S based on the acoustic wave A1 and the detection signal S based on the ultrasonic wave B2 from each other.

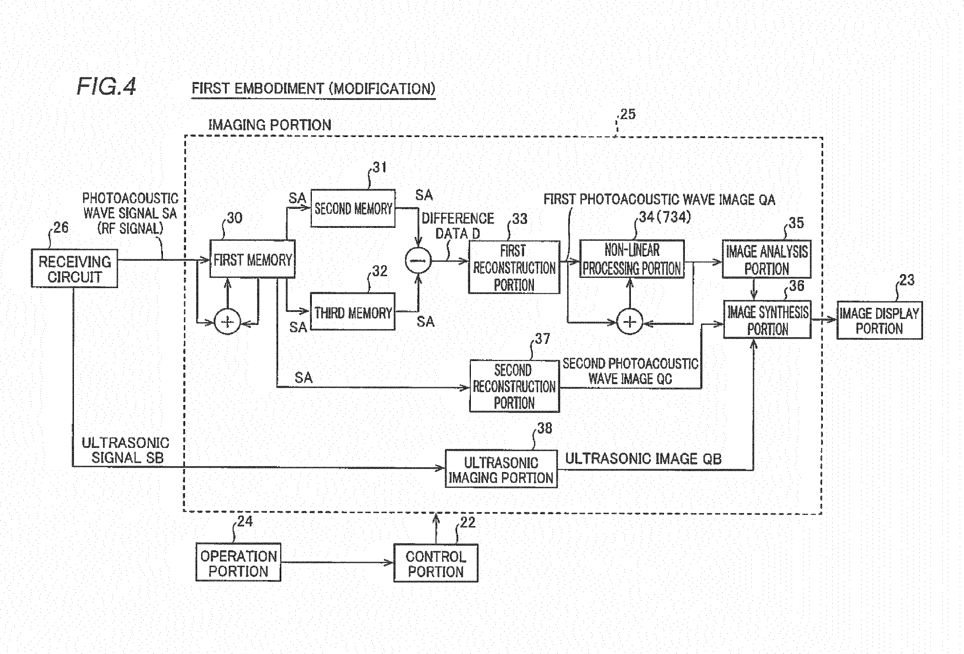

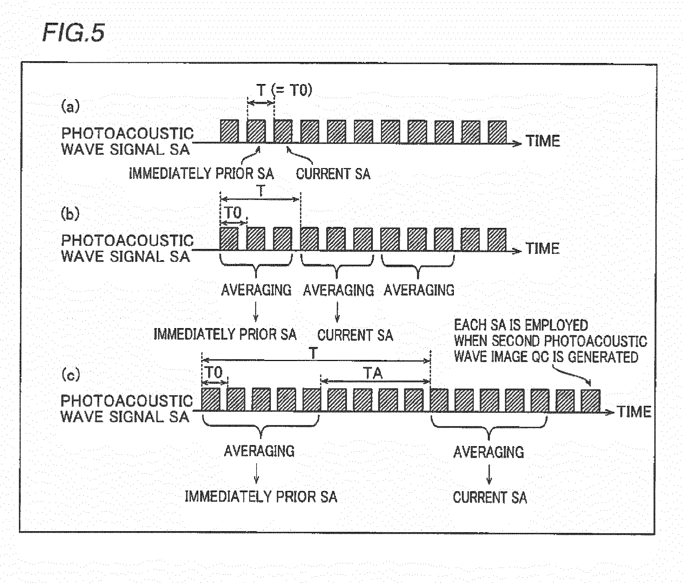

According to the first embodiment, the imaging portion 25 is configured to acquire the photoacoustic wave signal SA at a time interval T0, acquire difference data D by calculating a difference between a signal based on the acquired photoacoustic wave signal SA (current photoacoustic wave signal SA) and a signal based on a photoacoustic wave signal SA (immediately prior photoacoustic wave signal SA) acquired immediately prior to the acquired photoacoustic wave signal SA at a time interval T, and generate the first photoacoustic wave image QA on the basis of the acquired difference data D, as shown in Figs. 4 and 5. The time interval T0 is an example of the "first time interval" in the present invention. The time interval T is an example of the "third time interval" in the present invention.

According to the first embodiment, the photoacoustic imager 100 is configured to be capable of setting the time interval T (time interval T0) to at least 0.1 msec and not more than 100 msec. According to the first embodiment, the photoacoustic imager 100 is further configured to set the time interval T (time interval T0) to at least 1 msec and not more than 50 msec.

Specifically, the imaging portion 25 is provided with a first memory 30, a second memory 31, and a third memory 32, as shown in Fig. 4. The first memory 30 is configured to acquire the photoacoustic wave signal SA from the receiving circuit 26. The first memory 30 is further configured to store the acquired photoacoustic wave signal SA. The first memory 30 is also configured to average a prescribed number of photoacoustic wave signals SA.

The first memory 30 is configured to transmit the photoacoustic wave signal SA alternately to the second memory 31 and the third memory 32 (see Fig. 4) at the time interval T on the basis of a control signal from the control portion 22. More specifically, the first memory 30 is configured to transmit a subsequently acquired photoacoustic wave signal SA to the third memory 32 after the time interval T elapses following transmission of the acquired photoacoustic wave signal SA to the second memory 31 and to transmit a further subsequently acquired photoacoustic wave signal SA to the second memory 31 after the time interval T further elapses.

The first memory 30 is configured to be capable of changing the time interval T and the aforementioned prescribed number for averaging on the basis of a control signal from the control portion 22.

For example, view (a) of Fig. 5 illustrates an example in which the first memory 30 transmits the acquired photoacoustic wave signal SA alternately to the second memory 31 and the third memory 32 each time the first memory 30 acquires the photoacoustic wave signal SA from the receiving circuit 26 (at the time interval T0) without averaging (setting the prescribed number to 1). In this case, in view (a) of Fig. 5, no averaging is performed, and hence the time interval T can be set to a shorter time interval as compared with in view (b) and view (c) of Fig. 5 described later.

View (b) of Fig. 5 illustrates an example in which the first memory 30 averages three photoacoustic wave signals SA and transmits the averaged photoacoustic wave signal SA alternately to the second memory 31 and the third memory 32 each time the first memory 30 acquires three photoacoustic wave signals SA from the receiving circuit 26 (at the time interval T). In this case, in view (b) of Fig. 5, the photoacoustic wave signal SA can be transmitted to the second memory 31 and the third memory 32 in a state where the signal-noise ratio of the photoacoustic wave signal SA is improved by averaging, unlike in view (a) of Fig. 5. The averaged photoacoustic wave signal SA is an example of the "averaged signal" in the present invention.

View (c) of Fig. 5 illustrates an example in which the first memory 30 averages five photoacoustic wave signals SA and transmits the averaged photoacoustic wave signal SA alternately to the second memory 31 and the third memory 32 each time the first memory 30 acquires nine photoacoustic wave signals SA from the receiving circuit 26 (at the time interval T). The five photoacoustic wave signals SA to be averaged are five sequentially acquired photoacoustic wave signals SA of the aforementioned nine photoacoustic wave signals SA. In this case, in view (c) of Fig. 5, a time interval TA is generated between the immediately prior photoacoustic wave signal SA and the current photoacoustic wave signal SA, unlike in view (b) of Fig. 5. Thus, the time interval TA is generated so that the difference between the immediately prior photoacoustic wave signal SA and the current photoacoustic wave signal SA is increased, and hence the difference data D (first photoacoustic wave image QA) described later can be more clearly generated. The time interval TA is an example of the "second time interval" in the present invention.

The aforementioned prescribed number for averaging may be a number other than 3 and 5 and is preferably properly set on the basis of the velocity of the detection object Pa, the size of the noise components of the photoacoustic wave signals SA, etc. Each of the photoacoustic wave signals SA shown in Fig. 5 is transmitted to a second reconstruction portion 37 as described later and is employed when the second reconstruction portion 37 generates the second photoacoustic wave image QC.

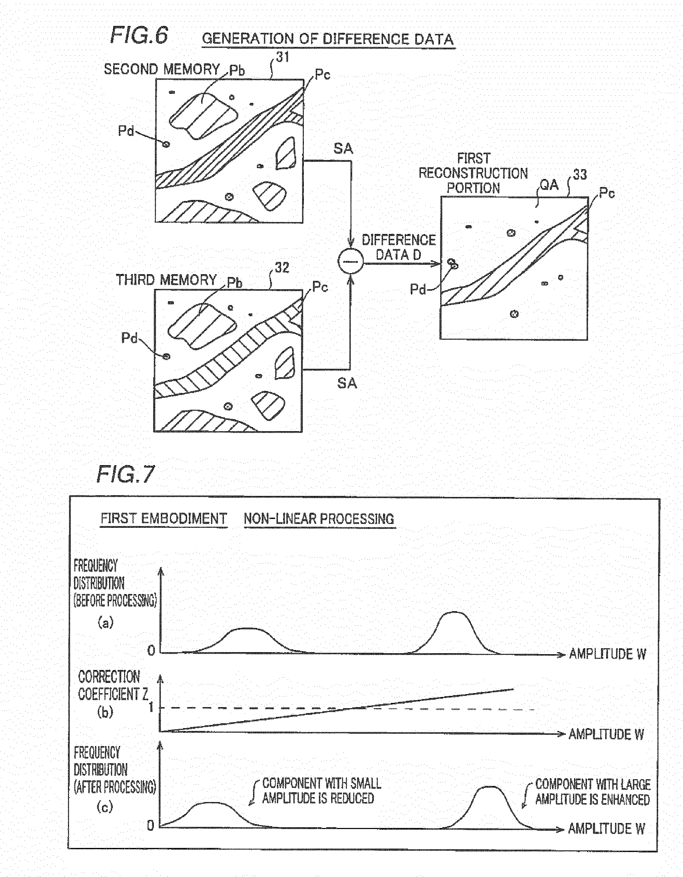

As shown in Fig. 6, the imaging portion 25 is configured to generate the difference data D by calculating a difference between the photoacoustic wave signal SA including the RF signal transmitted from the first memory 30 to the second memory 31 and the photoacoustic wave signal SA including the RF signal transmitted from the first memory 30 to the third memory 32 at the time interval T. In other words, the difference data D is obtained by calculating the difference between the signal based on the photoacoustic wave signal SA acquired by the imaging portion 25 and the signal based on a photoacoustic wave signal SA acquired immediately prior to the acquired photoacoustic wave signal SA. In Fig. 6, the photoacoustic wave signals SA stored in the second memory 31 and the third memory 32 are imaged for illustration purpose, but according to the first embodiment, the second memory 31 and the third memory 32 store the photoacoustic wave signals SA in the state of projection signals (see Fig. 3).

The imaging portion 25 is configured to calculate a signal difference value between the photoacoustic wave signal SA from the second memory 31 and the photoacoustic wave signal SA from the third memory 32 as difference calculation. For example, the imaging portion 25 calculates the signal difference value as X-Y or Y-X when setting the signal value of the photoacoustic wave signal SA from the second memory 31 at a certain coordinate point to X and the signal value of the photoacoustic wave signal SA from the third memory 32 at the corresponding coordinate point to Y.

The difference data D is generated in a state where data of an unmoving portion (an object Pb in Fig. 6, for example) of the detection object Pa is subtracted and data of a moving portion (blood Pc in Fig. 6, for example) of the detection object Pa remains. In other words, the difference data D is generated as data obtained by extracting the portions in which the intensities of the photoacoustic wave signals SA temporally change.

A first reconstruction portion 33 is configured to generate the first photoacoustic wave image QA indicating the detection object Pa in motion on the basis of the acquired difference data D. Specifically, the first reconstruction portion 33 is configured to reconstruct the difference data D configured as projection signals into the first photoacoustic wave image QA by processing performed by an analytical method (back projection processing performed by an FBP (filtered back projection) method or the like, for example). In other words, the first reconstruction portion 33 is configured to generate image data (first photoacoustic wave image QA) corresponding to the spatial position of the detection object Pa on the basis of information about the projection signals contained in the difference data D.

As shown in Fig. 4, the first reconstruction portion 33 is further configured to transmit the first photoacoustic wave image QA that is reconstructed and imaged to a non-linear processing portion 34.

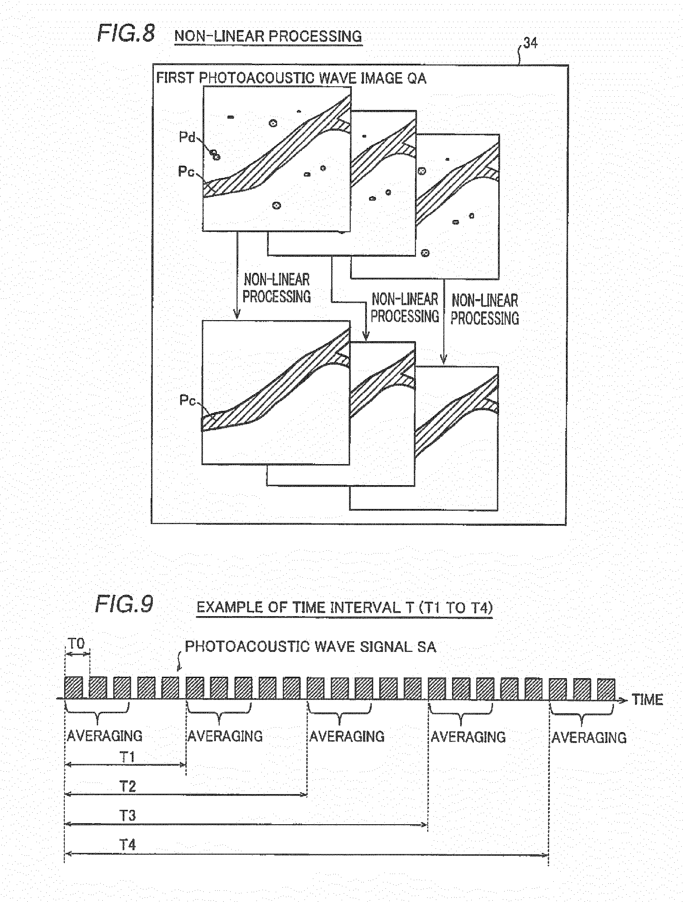

According to the first embodiment, the non-linear processing portion 34 is configured to perform processing for reducing a noise component contained in each of a plurality of first photoacoustic wave images QA reconstructed by the first reconstruction portion 33 and to perform non-linear processing for enhancing a signal component contained in each of the first photoacoustic wave images QA, as shown in Figs. 7 and 8. The imaging portion 25 is configured to synthesize the plurality of non-linearly processed first photoacoustic wave images QA.

For example, the non-linear processing portion 34 performs non-linear processing on the first photoacoustic wave image QA by multiplying a value of each piece of data of the first photoacoustic wave image QA by a correction coefficient Z ($0 \leq Z \leq 2$) expressed by the following formula (2), setting $\underline{a}$ as a function ($-1 \leq \underline{a} \leq +1$) of the amplitude W of the photoacoustic wave signal SA, as shown in Fig. 7.

$$Z = a (W) + 1 \dots (2)$$

View (a) of Fig. 7 illustrates an example of the frequency distribution of the photoacoustic wave signal SA. View (b) of Fig. 7 illustrates the correction coefficient Z expressed by the aforementioned formula (2). In this case, $\underline{a}$ (W) is set to have a relationship of a linear function with respect to the size of the amplitude W, for example. View (c) of Fig. 7 illustrates an example of the frequency distribution of the photoacoustic wave signal SA after multiplication of the correction coefficient Z (non-linear processing). In other words, the non-linear processing portion 34 performs processing for further increasing the signal intensity of a signal having a larger amplitude (for further increasing the amplitude) and performs processing for further reducing the signal intensity of a signal having a smaller amplitude (for further reducing the amplitude) according to the size of the amplitude that is the value of data of the first photoacoustic wave image QA.

Specifically, when the value of data of the first photoacoustic wave image QA is larger than a prescribed amplitude W ($\underline{a} > 1$), the signal intensity is increased, and when the value of data of the first photoacoustic wave image QA is smaller than the prescribed amplitude W ($\underline{a} < 1$), the signal intensity is reduced.

Thus, the non-linear processing portion 34 reduces the component of a signal having a small amplitude that generally serves as a noise component and enhances the component of a signal having a large amplitude that serves as a signal component in the first photoacoustic wave image QA. As shown in Fig. 8, the signal component (Pc, for example) in the first photoacoustic wave image QA is emphasized, and the noise component (Pd, for example) in the first photoacoustic wave image QA is emphasized is removed. Furthermore, an edge portion of the signal component in the first photoacoustic wave image QA is emphasized.

The non-linear processing portion 34 of the imaging portion 25 performs the aforementioned non-linear processing on each of the plurality of (three in Fig. 8) first photoacoustic wave images QA. The imaging portion 25 is configured to synthesize the plurality of non-linearly processed first photoacoustic wave images QA. Thus, the first photoacoustic wave images QA indicating the movement of the detection object Pa are synthesized, and hence a synthetic first photoacoustic wave image QA contains information about the locus of the movement of the detection object Pa. The non-linear processing portion 34 is configured to transmit the synthetic first photoacoustic wave image QA to an image analysis portion 35 (see Fig. 4) described later.

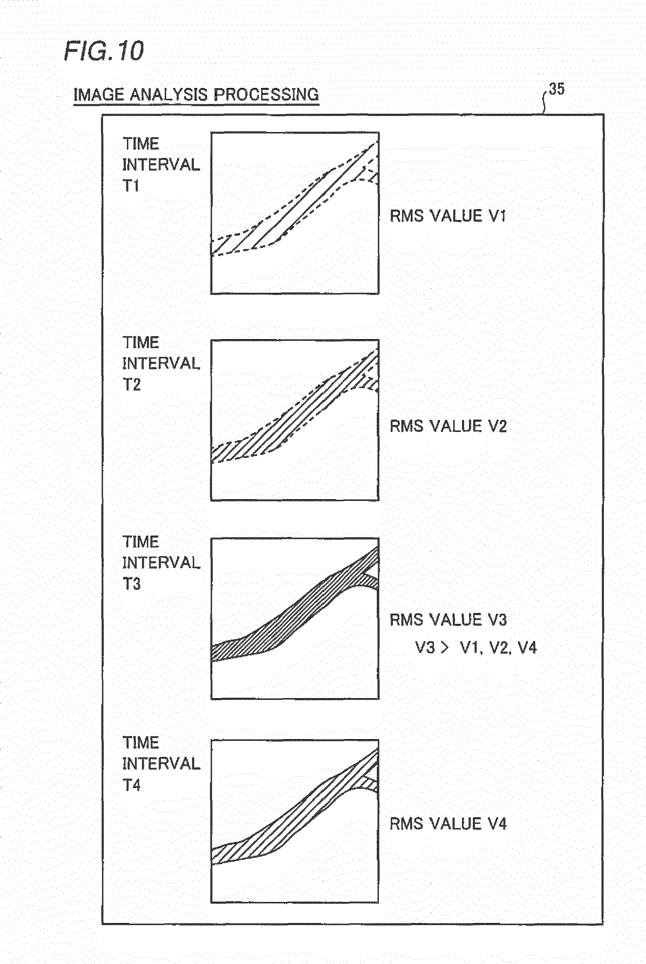

According to the first embodiment, the photoacoustic imager 100 is configured to be capable of setting a plurality of time intervals T (time intervals T1 to T4, for example) that are equal to or greater than the time interval T0 and are different from each other, as shown in Figs. 9 and 10. The imaging portion 25 is configured to generate first photoacoustic wave images QA corresponding to the respective time intervals T1 to T4, to select a first photoacoustic wave image QA having the highest image definition from the generated first photoacoustic wave images QA, and to output the selected first photoacoustic wave image QA to the image display portion 23. A more detailed description is provided below.

For example, assume that the time intervals T have a relationship of T1 < T2 < T3 < T4, as shown in Fig. 9. The imaging portion 25 generates the first photoacoustic wave images QA corresponding to the respective time intervals T1 to T4, as shown in Fig. 10. The imaging portion 25 is provided with the image analysis portion 35, and the image analysis portion 35 is configured to calculate RMS (root mean square) values V1 to V4 with respect to the first photoacoustic wave images QA corresponding to the respective time intervals T1 to T4. In other words, the image analysis portion 35 is configured to calculate an average value of the squares of values of pixels in the first photoacoustic wave image QA. If the moving detection object Pa is unclear, for example, the RMS value is small, and if the moving detection object Pa is clear, the RMS value is large.

When the RMS value V3 of the first photoacoustic wave image QA corresponding to the time interval T3 is the largest of the RMS values V1 to V4, for example, the image analysis portion 35 selects the first photoacoustic wave image QA corresponding to the time interval T3 as an image having the highest image definition and transmits the same to an image synthesis portion 36. The image analysis portion 35 transmits information indicating that the time interval T3 of the time intervals T is a time interval in which an image having the highest image definition is generated to the image synthesis portion 36 or the control portion 22.

According to the first embodiment, the image synthesis portion 36 of the imaging portion 25 is configured to superpose the first photoacoustic wave image QA generated on the basis of the difference data D and the second photoacoustic wave image QC acquired by imaging the photoacoustic wave signal SA or the ultrasonic image QB acquired by imaging the detection signal S based on the ultrasonic wave B2 and to output the same to the image display portion 23, as shown in Fig. 4. A more detailed description is provided below.

The imaging portion 25 is provided with the second reconstruction portion 37. The second reconstruction portion 37 is configured to acquire the photoacoustic wave signal SA from the first memory 30 and to reconstruct the acquired photoacoustic wave signal SA into the second photoacoustic wave image QC. In other words, the second photoacoustic wave image QC is an image indicating whether or not the detection object Pa exists in the specimen P, unlike the first photoacoustic wave image QA generated on the basis of the difference data D. The second reconstruction portion 37 is configured to transmit the generated second photoacoustic wave image QC to the image synthesis portion 36.

The imaging portion 25 is provided with an ultrasonic imaging portion 38. The ultrasonic imaging portion 38 is configured to acquire the ultrasonic signal SB from the receiving circuit 26 and to reconstruct the acquired ultrasonic signal SB into the ultrasonic image QB. In other words, the ultrasonic image QB is an image indicating whether or not the detection object Pa exists in the specimen P, unlike the first photoacoustic wave image QA generated on the basis of the difference data D. The ultrasonic imaging portion 38 is configured to transmit the generated ultrasonic image QB to the image synthesis portion 36.

The image synthesis portion 36 is configured to acquire the aforementioned first photoacoustic wave image QA, second photoacoustic wave image QC, and ultrasonic image QB and to generate a display image QD by synthesizing the acquired images on the basis of a command from the control portion 22. In other words, the image synthesis portion 36 is configured to be capable of displaying an image of the detection object Pa on the image display portion 23 by a desired image(s) and a desired image synthesis method selected by the operator.

Specifically, the control portion 22 is configured to transmit any of a control signal for outputting only the first photoacoustic wave image QA to the image display portion 23, a control signal for synthesizing the first photoacoustic wave image QA and the second photoacoustic wave image QC and outputting the synthetic image to the image display portion 23, a control signal for synthesizing the first photoacoustic wave image QA and the ultrasonic image QB and outputting the synthetic image to the image display portion 23, and a control signal for synthesizing the first photoacoustic wave image QA, the second photoacoustic wave image QC, and the ultrasonic image QB and outputting the synthetic image to the image display portion 23 to the imaging portion 25 (image synthesis portion 36) on the basis of an input operation of the operator through the operation portion 24.

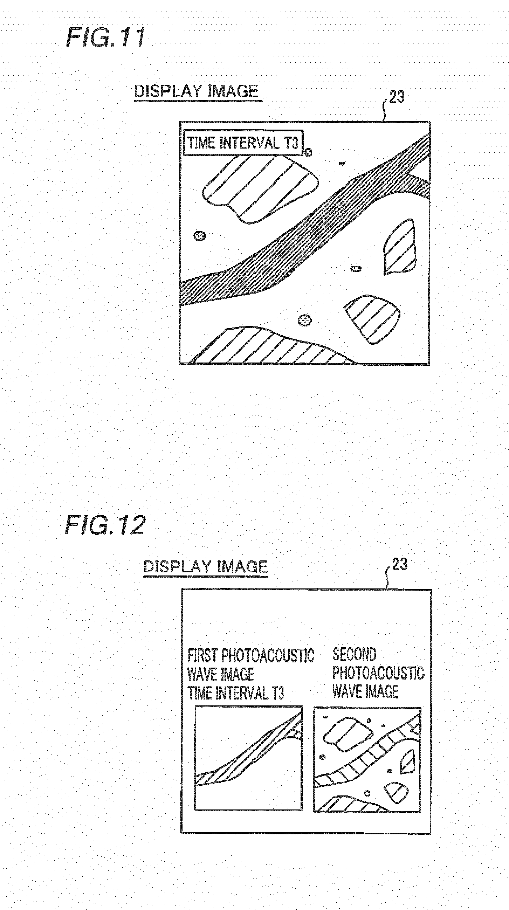

As shown in Fig. 11, the image synthesis portion 36 is configured to be capable of selecting whether to superpose a plurality of images and display one screen, as shown in Fig. 11 or to align the plurality of images and display one screen, as shown in Fig. 12, when generating the display image QD by synthesizing the images. For example, Fig. 11 shows a state where the display image QD obtained by synthesizing the first photoacoustic wave image QA and the second photoacoustic wave image QC to overlap each other is displayed on the image display portion 23. In this case, the first photoacoustic wave image QA is displayed in color (red color, for example) on the image display portion 23 while the second photoacoustic wave image QC is displayed in black and white on the image display portion 23, whereby the visibility can be further improved. Fig. 12 shows a state where the display image QD obtained by synthesizing the first photoacoustic wave image QA and the second photoacoustic wave image QC to display the same side by side is displayed on the image display portion 23.

The image synthesis portion 36 is further configured to output information indicating which of the plurality of time intervals T is a time interval in which the first photoacoustic wave image QA having the highest image definition can be generated (information indicating a time interval in which an image having the highest image definition is generated) together with the display image QD to the image display portion 23 when generating this display image QD by synthesizing the images in the case where the plurality of time intervals T (the time intervals T1 to T4, for example) are set.

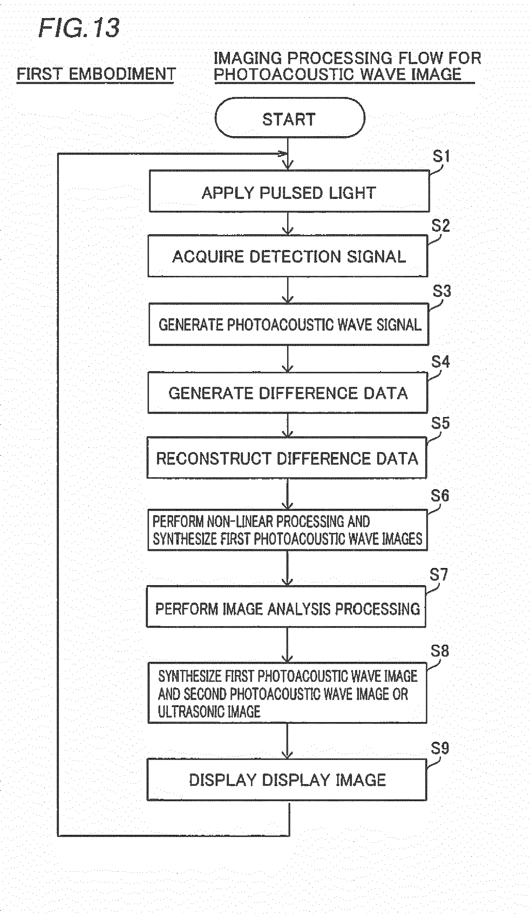

Imaging processing for photoacoustic wave images in the photoacoustic imager 100 according to the first embodiment is now described with reference to Fig. 13. Processing in the photoacoustic imager 100 is performed by the control portion 22 and the imaging portion 25.

First, the light source portion 11 applies pulsed light to the specimen P at a step S1. Then, the control portion 22 advances to a step S2.

At the step S2, the ultrasonic vibrator portion 12 detects the acoustic wave $\underline{A}$, and the detection signal S (see Fig. 2) is acquired. Then, the control portion 22 advances to a step S3.

At the step S3, the receiving circuit 26 generates the photoacoustic wave signal SA (see Fig. 3) on the basis of the detection signal S. Then, the control portion 22 advances to a step S4.

The imaging portion 25 generates the difference data D (see Fig. 7) on the basis of the photoacoustic wave signal SA at the step S4. Then, the control portion 22 advances to a step S5.

The first reconstruction portion 33 of the imaging portion 25 reconstructs the difference data D at the step S5 and generates the first photoacoustic wave image QA. Then, the control portion 22 advances to a step S6.

At the step S6, the non-linear processing portion 34 of the imaging portion 25 performs non-linear processing (Figs. 7 and 8) on the first photoacoustic wave image QA. The prescribed number of (three, for example) first photoacoustic wave images QA are synthesized. Then, the control portion 22 advances to a step S7.

At the step S7, the image analysis portion 35 performs image analysis processing (see Fig. 10) for selecting the first photoacoustic wave image QA having the highest image definition from the plurality of first photoacoustic wave images QA. Then, the control portion 22 advances to a step S8.

At the step S8, the image synthesis portion 36 synthesizes the first photoacoustic wave image QA and the second photoacoustic wave image QC or the ultrasonic image QB and generates the display image QD. Then, the control portion 22 advances to a step S9.

At the step S9, the image display portion 23 displays the display image QD (Figs. 11 and 12). Then, the control portion 22 returns to the step S1.

According to the first embodiment, the following effects can be obtained.

According to the first embodiment, as hereinabove described, the photoacoustic imager 100 is configured to acquire the difference data D of signals acquired on the basis of the plurality of photoacoustic wave signals SA detected at the different times of the photoacoustic wave signals SA, whereby the data of the unmoving portion of the detection object Pa is subtracted while the data of the moving portion of the detection object Pa remains. Therefore, the portions in which the intensities of the photoacoustic wave signals SA temporally change can be extracted. Thus, the first photoacoustic wave image QA (display image QD) indicating the detection object Pa in motion in the specimen P can be generated on the basis of the photoacoustic wave signals SA.

According to the first embodiment, as hereinabove described, the imaging portion 25 is configured to acquire the photoacoustic wave signal SA at the time interval T, to acquire the difference data D by calculating the difference between the signal based on the acquired photoacoustic wave signal SA and the signal based on the photoacoustic wave signal SA acquired immediately prior to the acquired photoacoustic wave signal SA, and to generate the first photoacoustic wave image QA on the basis of the acquired difference data D. Thus, the difference data D between the signal based on the acquired photoacoustic wave signal SA and the signal based on the photoacoustic wave signal SA acquired immediately prior to the acquired photoacoustic wave signal SA is acquired, and hence the difference data D can be easily acquired each time the photoacoustic wave signal SA is acquired. Furthermore, the photoacoustic wave signal SA is acquired at the time interval T, and hence the first photoacoustic wave image QA of the detection object Pa in the specimen P moved during the time interval T can be continuously repetitively generated.

According to the first embodiment, as hereinabove described, the time interval T can be set to at least 0.1 msec and not more than 100 msec. The blood flow velocity of the blood (detection object Pa) in the human body (specimen P) is generally at least 1 mm/s and not more than 1000 mm/s. The resolution of imaging of the photoacoustic imager 100 according to the first embodiment is within a range from several 10 $\mu$m order to several mm order. In consideration of this point, the time interval T can be set to at least 0.1 msec according to the first embodiment, and hence the moving distance of the aforementioned blood is at least 0.1 $\mu$m and not more than 100 $\mu$m. Thus, the blood having a relatively large blood flow velocity (blood flow velocity of 1000 mm/s, for example) can be observed in correspondence to the resolution of imaging of the photoacoustic imager 100. According to the first embodiment, the time interval T can be set to not more than 100 msec, and hence the moving distance of the aforementioned blood is at least 100 $\mu$m and not more than 100 mm. Thus, the blood having a relatively small blood flow velocity (blood flow velocity of 1 mm/s, for example) can be observed in correspondence to the resolution of imaging of the photoacoustic imager 100. Therefore, the time interval T is set to at least 0.1 msec and not more than 100 msec, whereby the first photoacoustic wave image QA indicating the movement of the blood in the human body can be properly generated in correspondence to the resolution of imaging of the photoacoustic imager 100.

According to the first embodiment, as hereinabove described, the time interval T is set to 1 msec and not more than 50 msec. Thus, the moving distance of the aforementioned blood is within a range from 1 $\mu$m to 1 mm when the time interval T is set to 1 msec, and the moving distance of the aforementioned blood is within a range from 50 $\mu$m to 50 mm when the time interval T is set to 50 msec, whereby the first photoacoustic wave image QA can be generated in closer correspondence to the resolution of imaging of the photoacoustic imager 100.

According to the first embodiment, as hereinabove described, the ultrasonic vibrator portion 12 and the receiving circuit 26 are configured to generate the photoacoustic wave signal SA including the RF signal (see Figs. 2 and 3) on the basis of the detected acoustic wave $\underline{A}$, and the imaging portion 25 is configured to generate the first photoacoustic wave image QA on the basis of the difference data D acquired on the basis of the plurality of RF signals (photoacoustic wave signals SA) detected at the different times of RF signals (photoacoustic wave signals SA). Generally, fine information (such as information indicating the phase of the signal) contained in the RF signal may be lost when the RF signal is demodulated (detected). According to the first embodiment, on the other hand, the first photoacoustic wave image QA is generated on the basis of the difference data D acquired on the basis of the plurality of RF signals detected at the different times of RF signals, and hence the first photoacoustic wave image QA (display image QD) can be generated without losing the fine information contained in the RF signal. Consequently, the first photoacoustic wave image QA faithfully indicating the movement of the detection object Pa can be generated.

According to the first embodiment, as hereinabove described, the imaging portion 25 is configured to acquire the photoacoustic wave signal SA at the time interval T, to set the plurality of time intervals T (time intervals T1 to T4) that are different from each other, to generate the first photoacoustic wave images QA corresponding to the plurality of respective time intervals T (time intervals T1 to T4), to select the first photoacoustic wave image QA having the highest image definition from the generated first photoacoustic wave images QA, and to output the selected first photoacoustic wave image QA to the image display portion 23. Thus, the operator (user) can visually recognize the first photoacoustic wave image QA with the highest image definition even when the time interval T in which the image definition becomes highest is varied according to the movement (such as the velocity) of the detection object Pa.

According to the first embodiment, as hereinabove described, the imaging portion 25 is configured to generate the plurality of first photoacoustic wave images QA, to perform non-linear processing for performing at least one of processing for reducing the noise component contained in each of the plurality of first photoacoustic wave images QA and processing for enhancing the signal component contained in each of the first photoacoustic wave images QA, and to synthesize the plurality of non-linearly processed first photoacoustic wave images QA. Thus, the first photoacoustic wave image QA can be generated while the signal component with respect to the noise component is increased in the first photoacoustic wave image QA by the non-linear processing. Furthermore, the plurality of non-linearly processed first photoacoustic wave images QA are synthesized, whereby the first photoacoustic wave image QA in which the locus of the movement of the detection object Pa is further emphasized can be generated.

According to the first embodiment, as hereinabove described, the ultrasonic vibrator portion 12 is configured to generate the ultrasonic wave B1 to be applied to the specimen P, to detect the ultrasonic wave B2 applied to the specimen P and reflected in the specimen P, and to generate the ultrasonic signal SB. Furthermore, the imaging portion 25 is configured to superpose the first photoacoustic wave image QA generated on the basis of the difference data D and at least one of the second photoacoustic wave image QC acquired by imaging the photoacoustic wave signal SA and the ultrasonic image QB acquired by imaging the ultrasonic detection signal and to output the same to the image display portion 23. Thus, at least one of the second photoacoustic wave image QC and the ultrasonic image QB that are images indicating whether or not the detection object Pa exists in the specimen P and the first photoacoustic wave image QA that is an image indicating the detection object Pa in motion are superposed to be displayed on the image display portion 23, and hence the operator (user) can visually recognize the position of the detection object Pa in the specimen P and the movement of the detection object Pa associated with each other.

According to the first embodiment, as hereinabove described, the light source portion 11 is provided with the semiconductor light-emitting elements 11a (any of light-emitting diode elements, semiconductor laser elements, and organic light-emitting diode elements). Thus, the semiconductor light-emitting elements 11a can apply light whose repetition frequency is relatively high (at least 1 kHz, for example), unlike a solid-state laser light source that applies pulsed light whose repetition frequency is about 10 Hz. Consequently, a time interval in which light is applied can be reduced, and hence the first photoacoustic wave image QA indicating the detection object that is traveling a long distance in a relatively short amount of time (whose moving velocity is large) can be also generated.

According to the first embodiment, as hereinabove described, the imaging portion 25 is configured to average the photoacoustic wave signals SA acquired at the time intervals T0, to acquire the difference data D by calculating the difference between the photoacoustic wave signal SA currently averaged and the photoacoustic wave signal SA averaged immediately prior to the currently averaged photoacoustic wave signal SA, and to generate the first photoacoustic wave image QA on the basis of the acquired difference data D. Thus, the difference data D can be acquired in the state where the signal-noise ratio of the photoacoustic wave signal SA is improved by averaging.

According to the first embodiment, as hereinabove described, the imaging portion 25 is configured such that the time interval TA equal to or greater than the time interval T0 is provided between a time point when the immediately prior averaged signal (averaged photoacoustic wave signal SA) is generated and a time point when the current averaged signal (averaged photoacoustic wave signal SA) is generated. Thus, the time interval TA is provided, and hence the difference between the immediately prior averaged signal and the current averaged signal can be increased. Therefore, the difference data D and the first photoacoustic wave image QA more clearly indicating the detection object Pa in motion can be generated.

In this case, the imaging portion 25 is preferably configured to perform non-linear processing that is processing for reducing the noise component contained in the first photoacoustic wave image QA and for enhancing the signal component contained in the first photoacoustic wave image QA by multiplying the value of each piece of data of the first photoacoustic wave image QA by the correction coefficient Z expressed by the aforementioned formula (2), setting the function expressing the amplitude W of the photoacoustic wave signal SA as a variable as a. When the amplitude W of the photoacoustic wave signal SA is small, the photoacoustic wave signal SA often becomes the noise component in the first photoacoustic wave image QA, and when the amplitude W of the photoacoustic wave signal SA is large, the photoacoustic wave signal SA often becomes the signal component in the first photoacoustic wave image QA. Focusing on this point, according to the first embodiment, by multiplying the value of each piece of data of the first photoacoustic wave image QA by the correction coefficient Z expressed by the aforementioned formula (2), the noise component contained in the first photoacoustic wave image QA can be effectively reduced while the signal component contained in the first photoacoustic wave image QA can be effectively enhanced.

(Second Embodiment)

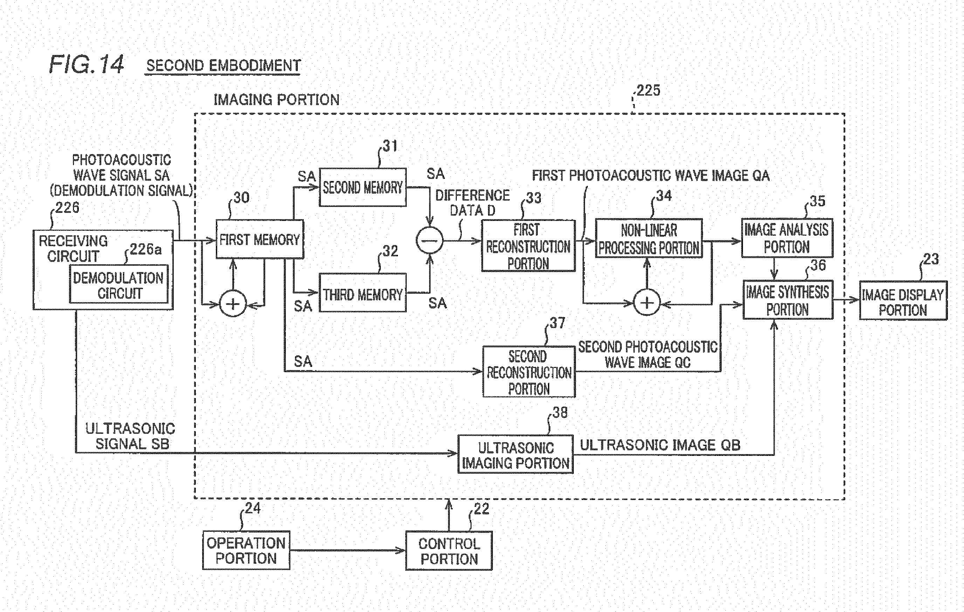

The structure of a photoacoustic imager 200 according to a second embodiment is now described with reference to Fig. 14. In this second embodiment, a receiving circuit is configured to generate a photoacoustic wave signal including a demodulation (detection) signal obtained by demodulating (detecting) an RF signal, unlike the photoacoustic imager according to the first embodiment in which the receiving circuit is configured to generate the photoacoustic wave signal including the RF signal. Portions of the photoacoustic imager 200 similar to those of the photoacoustic imager 100 according to the aforementioned first embodiment are denoted by the same reference numerals as those in the first embodiment, and redundant description is omitted.

As shown in Fig. 14, the photoacoustic imager 200 according to the second embodiment is provided with a receiving circuit 226. The receiving circuit 226 includes a demodulation (detector) circuit 226a. The demodulation circuit 226a is configured to demodulate (detect) a detection signal S including an RF signal acquired from an ultrasonic vibrator portion 12. For example, the demodulation circuit 226a acquires the demodulation signal that is a signal of an envelope component (excluding a modulation component or the like) in the waveform of the RF signal. The demodulation circuit 226a is further configured to transmit a photoacoustic wave signal SA including the demodulation signal to an imaging portion 225, similarly to the photoacoustic imager 100 according to the first embodiment. The imaging portion 225 is configured to generate a first photoacoustic wave image QA on the basis of difference data D acquired on the basis of a plurality of photoacoustic wave signals SA including demodulation signals detected at different times of photoacoustic wave signals SA including demodulation signals.

The remaining structures of the photoacoustic imager 200 according to the second embodiment are similar to those of the photoacoustic imager 100 according to the first embodiment.

According to the second embodiment, the following effects can be obtained.

According to the second embodiment, as hereinabove described, the ultrasonic vibrator portion 12 is configured to generate the detection signal S including the FR signal on the basis of a detected acoustic wave A, and the demodulation circuit 226a of the receiving circuit 226 is configured to generate the photoacoustic wave signal SA including the demodulation signal obtained by demodulating the RF signal. Furthermore, the imaging portion 225 is configured to generate the first photoacoustic wave image QA on the basis of the difference data D acquired on the basis of the plurality of photoacoustic wave signals SA including the demodulation signals detected at the different times of the photoacoustic wave signals SA including the demodulation signals. Thus, the data capacity of the demodulation signal is smaller than that of the RF signal, and hence the capacity of the difference data can be reduced. Consequently, an increase in the capacity of memories (a first memory 30, a second memory 31, and a third memory 32) of the imaging portion 225 for storing the difference data can be significantly reduced or prevented.

The remaining effects of the photoacoustic imager 200 according to the second embodiment are similar to those of the photoacoustic imager 100 according to the first embodiment.

(Third Embodiment)

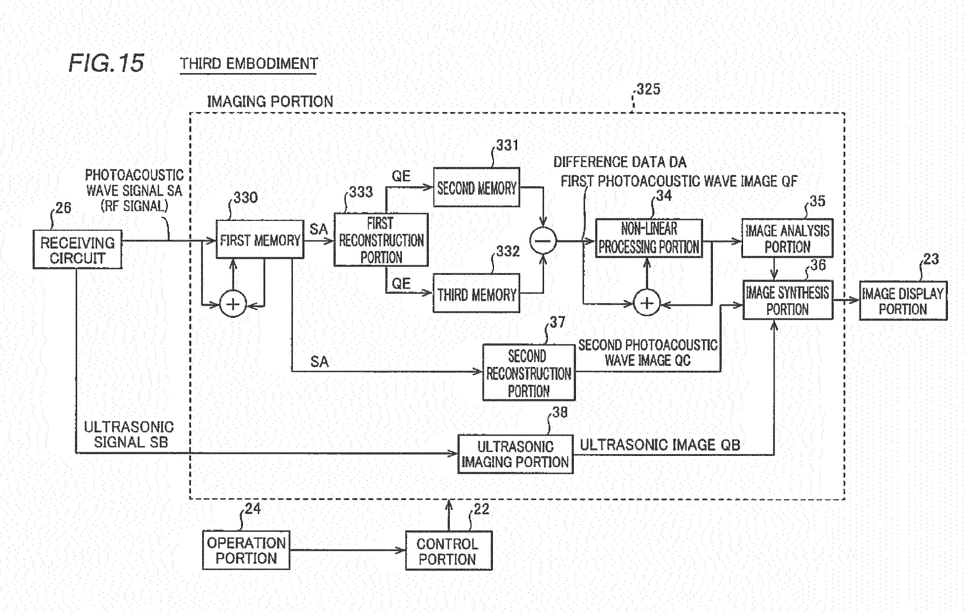

The structure of a photoacoustic imager 300 according to a third embodiment is now described with reference to Fig. 15. In the third embodiment, an imaging portion generates difference data after a first reconstruction portion reconstructs a photoacoustic wave signal, unlike the photoacoustic imager according to the first embodiment in which the first reconstruction portion reconstructs the difference data after the imaging portion generates the difference data.

As shown in Fig. 15, the photoacoustic imager 300 according to the third embodiment is provided with an imaging portion 325. The imaging portion 325 includes a first memory 330, a second memory 331, a third memory 332, and a first reconstruction portion 333. According to the third embodiment, the imaging portion 325 is configured to generate difference data DA after the first reconstruction portion 333 reconstructs a photoacoustic wave signal SA.

Specifically, the first memory 330 is configured to transmit the photoacoustic wave signal SA acquired from a receiving circuit 26 to the first reconstruction portion 333. The first reconstruction portion 333 is configured to transmit a photoacoustic wave image QE alternately to the second memory 331 and the third memory 332 at a time interval T after reconstructing the photoacoustic wave signal SA and generating the photoacoustic wave image QE. The second memory 331 and the third memory 332 each are configured to store the photoacoustic wave image QE.

The imaging portion 325 is further configured to retrieve the photoacoustic wave image QE from each of the second memory 331 and the third memory 332 and generate a first photoacoustic wave image QF including the difference data DA of the photoacoustic wave image QE. The first photoacoustic wave image QF including the difference data DA of the photoacoustic wave image QE is transmitted to a non-linear processing portion 34. The remaining processing is similar to that performed by the photoacoustic imager 100 according to the first embodiment.

The remaining structures of the photoacoustic imager 300 according to the third embodiment are similar to those of the photoacoustic imager 100 according to the first embodiment.

According to the third embodiment, the following effects can be obtained.

According to the third embodiment, as hereinabove described, the imaging portion 325 is configured to store the photoacoustic wave signal SA (photoacoustic wave image QE) reconstructed after the first reconstruction portion 333 reconstructs the photoacoustic wave signal SA in each of the second memory 331 and the third memory 332 and to generate the difference data DA on the basis of the reconstructed photoacoustic wave signal SA retrieved from each of the second memory 331 and the third memory 332. In general, data capacity after reconstruction is smaller than data capacity before reconstruction. The photoacoustic imager 300 is configured as described above, whereby the capacity of the photoacoustic wave signal SA (photoacoustic wave image QE) stored in each of the second memory 331 and the third memory 332 is reduced, and hence increases in the sizes of the second memory 331 and the third memory 332 can be significantly reduced or prevented.

The remaining effects of the photoacoustic imager 300 according to the third embodiment are similar to those of the photoacoustic imager 100 according to the first embodiment.

(Fourth Embodiment)

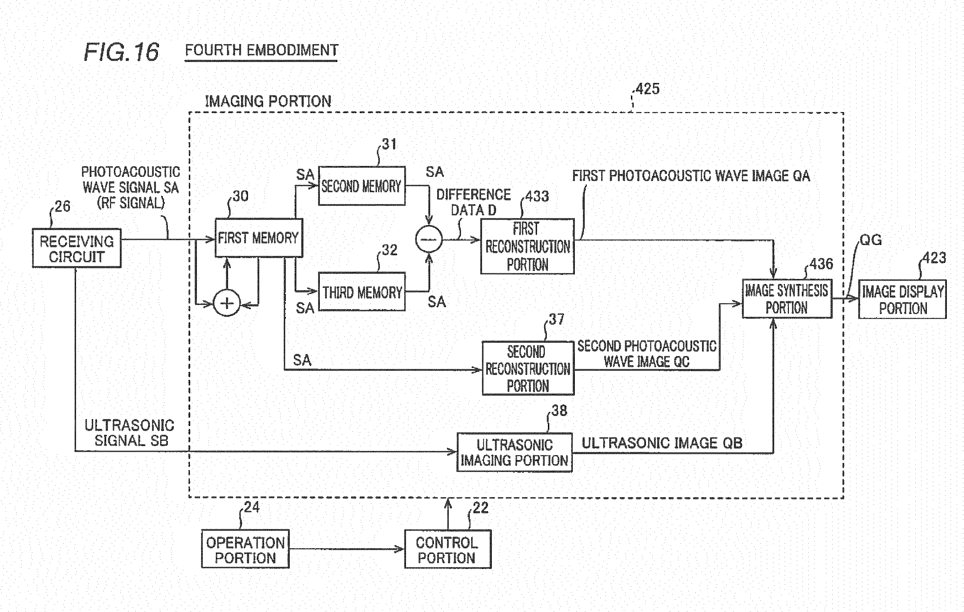

The structure of a photoacoustic imager 400 according to a fourth embodiment is now described with reference to Fig. 16. In the fourth embodiment, an imaging portion is configured to output first photoacoustic wave images generated on the basis of difference data and not synthesized to an image display portion at prescribed time intervals, unlike the photoacoustic imager according to the first embodiment in which the synthetic first photoacoustic wave image generated on the basis of the difference data by the imaging portion is output to the image display portion.

As shown in Fig. 16, the photoacoustic imager 400 according to the fourth embodiment is provided with an imaging portion 425 and an image display portion 423. The imaging portion 425 includes a first reconstruction portion 433 and an image synthesis portion 436 but does not include the non-linear processing portion 34 and the image synthesis portion 35 included in the imaging portion 25 according to the first embodiment.

According to the fourth embodiment, the imaging portion 425 is configured to output first photoacoustic wave images QA generated on the basis of difference data D and not synthesized to the image display portion 423 at time intervals T. In other words, the first reconstruction portion 433 transmits the first photoacoustic wave images QA to the image synthesis portion 436 at the time intervals T after generating the first photoacoustic wave images QA on the basis of the difference data D at the time intervals T. The image synthesis portion 436 is configured to generate a display image QG by synthesizing a first photoacoustic wave image QA and a second photoacoustic wave image QC or an ultrasonic image QB buy not synthesizing the first photoacoustic wave images QA. The image synthesis portion 436 is configured to transmit the display image QG to the image display portion 423 at a time interval T. The image display portion 423 is configured to update and display the display image QG at the time interval T. The time interval T is an example of the "fourth time interval" in the present invention.

The remaining structures of the photoacoustic imager 400 according to the fourth embodiment are similar to those of the photoacoustic imager 100 according to the first embodiment.

According to the fourth embodiment, the following effects can be obtained.

According to the fourth embodiment, as hereinabove described, the imaging portion 425 is configured to output the first photoacoustic wave images QA generated on the basis of the difference data D and not synthesized to the image display portion 423 at the time intervals T. Thus, no processing for synthesizing the first photoacoustic wave images QA is performed, and hence a processing load on the imaging portion 425 can be reduced.

The remaining effects of the photoacoustic imager 400 according to the fourth embodiment are similar to those of the photoacoustic imager 100 according to the first embodiment.

The embodiments disclosed this time must be considered as illustrative in all points and not restrictive. The range of the present invention is shown not by the above description of the embodiments but by the scope of claims for patent, and all modifications within the meaning and range equivalent to the scope of claims for patent are further included.

For example, while the signal difference value (X-Y or Y-X) between the photoacoustic wave signal from the second memory and the photoacoustic wave signal from the third memory is calculated as difference calculation according to the present invention in each of the aforementioned first to fourth embodiments, the present invention is not restricted to this. According to the present invention, difference calculation may alternatively be performed by a method other than calculation of the signal difference value between the photoacoustic wave signal from the second memory and the photoacoustic wave signal from the third memory. For example, a photoacoustic imager may be configured to perform processing (Y/X - 1) for subtracting 1 from a ratio (Y/X) of the photoacoustic wave signal from the third memory to the photoacoustic wave signal from the second memory as difference calculation.

While the difference data is acquired by calculating the difference between the acquired photoacoustic wave signal and the photoacoustic wave signal acquired immediately prior to the acquired photoacoustic wave signal in each of the aforementioned first to fourth embodiments, the present invention is not restricted to this. According to the present invention, the difference data may alternatively be acquired by calculating a difference between the acquired photoacoustic wave signal and a photoacoustic wave signal acquired other than immediately prior to the acquired photoacoustic wave signal. For example, the difference data may be acquired by calculating a difference between the acquired photoacoustic wave signal and a photoacoustic wave signal acquired prior to a previous prescribed time interval.

While the prescribed time interval according to the present invention is set to at least 1 msec and not more than 50 msec in each of the aforementioned first to fourth embodiments, the present invention is not restricted to this. According to the present invention, the prescribed time interval may alternatively be set to at least 1 msec and not more than 50 msec. For example, the prescribed time interval may be set to at least 0.1 msec and less than 1 msec or more than 50 msec and not more than 100 msec.

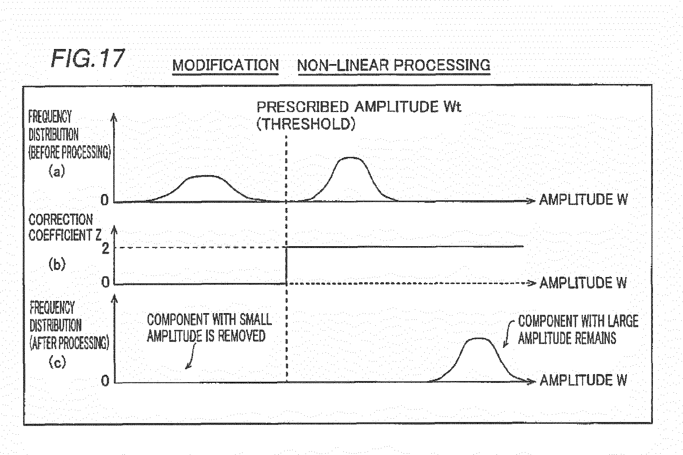

While processing for reducing the noise component contained in the first photoacoustic wave image or processing for enhancing the signal component contained in the first photoacoustic wave image is performed as the non-linear processing according to the present invention by multiplying a data value of the first photoacoustic wave image by the correction coefficient having a relationship of a linear function with the amplitude of the photoacoustic wave signal in each of the aforementioned first to fourth embodiments, the present invention is not restricted to this. According to the present invention, processing for reducing the noise component contained in the first photoacoustic wave image or processing for enhancing the signal component contained in the first photoacoustic wave image may alternatively be performed by multiplying the data value of the first photoacoustic wave image by a correction coefficient having no relationship of a linear function with the amplitude of the photoacoustic wave signal. For example, as in a modification shown in Figs. 4 and 17, a noise component contained in a first photoacoustic wave image may be reduced by processing performed by a threshold method.

Processing performed by a non-linear processing portion 734 according to the modification with the threshold method is processing for removing a component with an amplitude not larger than a prescribed amplitude Wt of data values of the first photoacoustic wave image (reducing the component to zero), as shown in Figs. 4 and 17. Thus, the non-linear processing portion 734 is configured to reduce the noise component of the first photoacoustic wave image.

For example, as shown in Fig. 17, the non-linear processing portion 734 performs non-linear processing on a first photoacoustic wave image QA by multiplying a value of each piece of data of the first photoacoustic wave image QA by a correction coefficient Z expressed by a following formula (3), setting $\underline{a}$ as a function ($-1 \leq \underline{a} \leq +1$) of the amplitude W of a photoacoustic wave signal SA. In other words, the following formula (3) expresses that a = 1 when amplitude W $\geq$ prescribed amplitude Wt and a = -1 when amplitude W < prescribed amplitude Wt in the aforementioned formula (2).

$$Z = 2 \ (W \geq Wt), \ Z = 0 \ (W < Wt) \ \dots \ (3)$$

While the imaging portion according to the present invention is configured to be capable of generating all of the first photoacoustic wave image, the second photoacoustic wave image, and the ultrasonic image in each of the aforementioned first to fourth embodiments, the present invention is not restricted to this. According to the present invention, it is only required to configure the imaging portion to be capable of generating at least the first photoacoustic wave image.

While the FBP method is employed as a method for reconstruction according to the present invention in each of the aforementioned first to fourth embodiments, the present invention is not restricted to this. According to the present invention, reconstruction may alternatively be performed by a method other than the FBP method. Reconstruction may be performed by a phasing addition method, a two-dimensional Fourier transform method, or the like, for example.

While the example (see Figs. 11 and 12) of displaying the time interval itself on the image display portion is shown as an example of being capable of visually recognizing the information indicating the time interval in which an image having the highest image definition is generated according to the present invention in each of the aforementioned first to fourth embodiments, the present invention is not restricted to this. According to the present invention, the information indicating the time interval in which an image having the highest image definition is generated may alternatively be visually recognized by displaying other than the time interval itself on the image display portion. For example, an index derived from the time interval may be capable of being visually recognized by number or color.

While the processing operations performed by the control portion according to the present invention are described, using the flowcharts described in a flow-driven manner in which processing is performed in order along a processing flow for the convenience of illustration in each of the aforementioned first to fourth embodiments, the present invention is not restricted to this. According to the present invention, the processing operations performed by the control portion may alternatively be performed in an event-driven manner in which processing is performed on an event basis. In this case, the processing operations performed by the control portion may be performed in a complete event-driven manner or in a combination of an event-driven manner and a flow-driven manner.

**Claims**

1. A photoacoustic imager (100, 200, 300, 400) comprising:

   a light source portion (11) that applies light to a specimen;
   a detection portion (12, 26, 226) that detects an acoustic wave generated by absorption of the light applied from the light source portion to the specimen by a detection object in the specimen and generates photoacoustic wave signals; and
   an imaging portion (25, 225, 325, 425) that generates a photoacoustic wave image indicating the detection object in motion by acquiring difference data of signals acquired on the basis of a plurality of the photoacoustic wave signals detected at different times of the photoacoustic wave signals to extract portions in which intensities of the photoacoustic wave signals temporally change.

2. The photoacoustic imager according to claim 1, wherein
   the imaging portion is configured to acquire the photoacoustic wave signals at first time intervals, to acquire the difference data by calculating differences between signals based on the photoacoustic wave signals that are acquired and signals based on the photoacoustic wave signals that have been acquired immediately prior to the photoacoustic wave signals that are acquired, and to generate the photoacoustic wave image on the basis of the difference data that is acquired.

3. The photoacoustic imager according to claim 2, wherein
   the imaging portion is configured to generate an averaged signal by averaging the photoacoustic wave signals acquired at the first time intervals, and is configured to acquire the difference data by calculating a difference between a current averaged signal and an immediately prior averaged signal and to generate the photoacoustic wave image on the basis of the difference data that is acquired.

4. The photoacoustic imager according to claim 3, wherein
   the imaging portion is configured such that a second time interval equal to or greater than each of the first time intervals is provided between a time point when the immediately prior averaged signal is generated and a time point when the current averaged signal is generated.

5. The photoacoustic imager according to any of claims 2 to 4, wherein
   each of the first time intervals is at least 0.1 msec and not more than 100 msec.

6. The photoacoustic imager according to claim 5, wherein
   each of the first time intervals is at least 1 msec and not more than 50 msec.

7. The photoacoustic imager according to any of claims 1 to 6, wherein
   the detection portion is configured to generate the photoacoustic wave signals including RF signals on the basis of the acoustic wave that is detected, and
   the imaging portion is configured to generate the photoacoustic wave image on the basis of the difference data acquired on the basis of a plurality of the RF signals detected at different times of the RF signals.

8. The photoacoustic imager according to any of claims 1 to 6, wherein
   the detection portion is configured to generate RF signals on the basis of the acoustic wave that is detected and to generate the photoacoustic wave signals including demodulation signals obtained by demodulating the RF signals, and
   the imaging portion is configured to generate the photoacoustic wave image on the basis of the difference data acquired on the basis of a plurality of the demodulation signals detected at different times of the demodulation signals.

9. The photoacoustic imager according to any of claims 1 to 8, further comprising a display portion (23, 423) that displays the photoacoustic wave image, wherein
   the imaging portion is configured to acquire the photoacoustic wave signals at first time intervals, to set a plurality of third time intervals that are equal to or greater than the first time intervals and are different from each other, to generate photoacoustic wave images corresponding to the plurality of respective third time intervals, to select the photoacoustic wave image having the highest image definition from the photoacoustic wave images that are generated, and to output the photoacoustic wave image that is selected to the display portion.

**10.** The photoacoustic imager according to any of claims 1 to 9, wherein
the imaging portion is configured to generate a plurality of photoacoustic wave images, to perform non-linear processing for performing at least one of processing for reducing a noise component contained in each of the plurality of photoacoustic wave images and processing for enhancing a signal component contained in each of the plurality of photoacoustic wave images, and to synthesize the plurality of photoacoustic wave images that are non-linearly processed.

**11.** The photoacoustic imager according to claim 10, wherein
the imaging portion is configured to perform the non-linear processing that is the processing for reducing the noise component contained in each of the photoacoustic wave images and processing for enhancing the signal component contained in each of the photoacoustic wave images by multiplying a value of each piece of data of the photoacoustic wave image by a correction coefficient Z expressed by a following formula (1), $Z = a (W) + 1$ ... (1), setting a function expressing an amplitude W of a photoacoustic wave signal as a variable as a.

**12.** The photoacoustic imager according to any of claims 1 to 9, further comprising a display portion (23, 423) that displays the photoacoustic wave image, wherein
the imaging portion is configured to output the photoacoustic wave image generated on the basis of the difference data and not synthesized to the display portion at a fourth time interval.

**13.** The photoacoustic imager according to any of claims 1 to 12, further comprising a display portion (23, 423) that displays the photoacoustic wave image, wherein
the detection portion is configured to generate an ultrasonic wave to be applied to the specimen, to detect the ultrasonic wave applied to the specimen and reflected in the specimen, and to generate an ultrasonic detection signal, and
the imaging portion is configured to superpose a first photoacoustic wave image generated on the basis of the difference data and at least one of a second photoacoustic wave image acquired by imaging a photoacoustic wave signal and an ultrasonic image acquired by imaging the ultrasonic detection signal and to output a superposed image to the display portion.

**14.** The photoacoustic imager according to any of claims 1 to 13, wherein
the light source portion includes any of a light-emitting diode element (11a), a semiconductor laser element (11a), and an organic light-emitting diode element (11a).

**15.** A photoacoustic imaging method comprising steps of:

applying light from a light source portion (11) to a specimen;
detecting an acoustic wave generated by absorption of the light applied from the light source portion to the specimen by a detection object in the specimen and generating photoacoustic wave signals; and
generating a photoacoustic wave image indicating the detection object in motion by acquiring difference data of signals acquired on the basis of a plurality of the photoacoustic wave signals detected at different times of the photoacoustic wave signals to extract portions in which intensities of the photoacoustic wave signals temporally change.

## FIG.1

FIRST EMBODIMENT

*FIG.2*

ACQUISITION OF
DETECTION SIGNAL S

M: SAMPLE NUMBER

N: NUMBER OF CHANNELS OF
PIEZOELECTRIC ELEMENTS

*FIG.3*

RECEIVING CIRCUIT

DETECTION SIGNAL S

PHOTOACOUSTIC WAVE SIGNAL SA

t (TIME)

FIG.4   FIRST EMBODIMENT (MODIFICATION)

IMAGING PORTION                                                                25

PHOTOACOUSTIC
WAVE SIGNAL SA
(RF SIGNAL)

26 RECEIVING CIRCUIT

30 FIRST MEMORY

31 SECOND MEMORY

32 THIRD MEMORY

SA

DIFFERENCE DATA D

33 FIRST RECONSTRUCTION PORTION

FIRST PHOTOACOUSTIC WAVE IMAGE QA

34 (734) NON-LINEAR PROCESSING PORTION

35 IMAGE ANALYSIS PORTION

36 IMAGE SYNTHESIS PORTION

23 IMAGE DISPLAY PORTION

37 SECOND RECONSTRUCTION PORTION

SECOND PHOTOACOUSTIC WAVE IMAGE QC

38 ULTRASONIC IMAGING PORTION

ULTRASONIC IMAGE QB

ULTRASONIC SIGNAL SB

24 OPERATION PORTION

22 CONTROL PORTION

EP 3 011 897 A1

FIG.5

(a)

T (= T0)

PHOTOACOUSTIC
WAVE SIGNAL SA ————————————————————→ TIME

IMMEDIATELY PRIOR SA    CURRENT SA

(b)

T
T0

PHOTOACOUSTIC
WAVE SIGNAL SA ————————————————————→ TIME

AVERAGING   AVERAGING   AVERAGING

IMMEDIATELY PRIOR SA    CURRENT SA

EACH SA IS EMPLOYED
WHEN SECOND PHOTOACOUSTIC
WAVE IMAGE QC IS GENERATED

(c)

T
T0        TA

PHOTOACOUSTIC
WAVE SIGNAL SA ————————————————————→ TIME

AVERAGING               AVERAGING

IMMEDIATELY PRIOR SA    CURRENT SA

FIG.6 GENERATION OF DIFFERENCE DATA

FIG.7

FIG.8 NON-LINEAR PROCESSING

FIG.9 EXAMPLE OF TIME INTERVAL T (T1 TO T4)

# FIG.10

IMAGE ANALYSIS PROCESSING 35

TIME
INTERVAL
T1

RMS VALUE V1

TIME
INTERVAL
T2

RMS VALUE V2

TIME
INTERVAL
T3

RMS VALUE V3

V3 > V1, V2, V4

TIME
INTERVAL
T4

RMS VALUE V4

*FIG.11*

DISPLAY IMAGE 23

TIME INTERVAL T3

*FIG.12*

DISPLAY IMAGE 23

FIRST PHOTOACOUSTIC WAVE IMAGE TIME INTERVAL T3

SECOND PHOTOACOUSTIC WAVE IMAGE

## FIG.13

FIRST EMBODIMENT　　　IMAGING PROCESSING FLOW FOR
　　　　　　　　　　　　　PHOTOACOUSTIC WAVE IMAGE

START

S1　APPLY PULSED LIGHT

S2　ACQUIRE DETECTION SIGNAL

S3　GENERATE PHOTOACOUSTIC WAVE SIGNAL

S4　GENERATE DIFFERENCE DATA

S5　RECONSTRUCT DIFFERENCE DATA

S6　PERFORM NON-LINEAR PROCESSING AND
SYNTHESIZE FIRST PHOTOACOUSTIC WAVE IMAGES

S7　PERFORM IMAGE ANALYSIS PROCESSING

S8　SYNTHESIZE FIRST PHOTOACOUSTIC WAVE IMAGE
AND SECOND PHOTOACOUSTIC WAVE IMAGE OR
ULTRASONIC IMAGE

S9　DISPLAY DISPLAY IMAGE

FIG.14 SECOND EMBODIMENT

FIG.15    THIRD EMBODIMENT

IMAGING PORTION                                                                325

DIFFERENCE DATA DA
FIRST PHOTOACOUSTIC WAVE IMAGE QF

PHOTOACOUSTIC
WAVE SIGNAL SA
(RF SIGNAL)

26
RECEIVING
CIRCUIT

330
FIRST MEMORY

SA

333
FIRST
RECONSTRUCTION
PORTION

QE

331
SECOND MEMORY

QE

332
THIRD MEMORY

34
NON-LINEAR
PROCESSING PORTION

35
IMAGE ANALYSIS
PORTION

36
IMAGE SYNTHESIS
PORTION

23
IMAGE DISPLAY
PORTION

SA

37
SECOND
RECONSTRUCTION
PORTION

SECOND PHOTOACOUSTIC
WAVE IMAGE QC

ULTRASONIC
SIGNAL SB

38
ULTRASONIC
IMAGING PORTION

ULTRASONIC IMAGE QB

24
OPERATION
PORTION

22
CONTROL
PORTION

EP 3 011 897 A1

*FIG.16*  FOURTH EMBODIMENT

IMAGING PORTION 425

PHOTOACOUSTIC
WAVE SIGNAL SA
(RF SIGNAL)

RECEIVING
CIRCUIT 26

FIRST MEMORY 30

SECOND MEMORY 31

THIRD MEMORY 32

SA

SA

DIFFERENCE
DATA D

FIRST
RECONSTRUCTION
PORTION 433

FIRST PHOTOACOUSTIC WAVE IMAGE QA

SECOND
RECONSTRUCTION
PORTION 37

SECOND PHOTOACOUSTIC
WAVE IMAGE QC

IMAGE SYNTHESIS
PORTION 436

QG

IMAGE DISPLAY
PORTION 423

ULTRASONIC
SIGNAL SB

ULTRASONIC
IMAGING PORTION 38

ULTRASONIC IMAGE QB

OPERATION
PORTION 24

CONTROL
PORTION 22

## FIG.17

MODIFICATION   NON-LINEAR PROCESSING

FREQUENCY DISTRIBUTION (BEFORE PROCESSING) (a)

PRESCRIBED AMPLITUDE Wt (THRESHOLD)

AMPLITUDE W

CORRECTION COEFFICIENT Z (b)

2

AMPLITUDE W

FREQUENCY DISTRIBUTION (AFTER PROCESSING) (c)

COMPONENT WITH SMALL AMPLITUDE IS REMOVED

COMPONENT WITH LARGE AMPLITUDE REMAINS

AMPLITUDE W

Europäisches
Patentamt

European
Patent Office

Office européen
des brevets

# EUROPEAN SEARCH REPORT

Application Number

EP 15 18 6402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2010/049049 A1 (ASAO YASUFUMI [JP] ET AL) 25 February 2010 (2010-02-25)<br>* paragraphs [0026], [0044], [0046] - [0064], [0070] - [0084]; figures 1-4,8,9a-9f * | 1,8,14, 15<br><br>12,13 | INV.<br>A61B5/00<br>A61B8/08 |
| X | WO 2014/063005 A1 (UNIV WASHINGTON) 24 April 2014 (2014-04-24)<br>* abstract; figures 27,28 *<br>& US 2015/245771 A1 (WANG LIHONG V [US] ET AL) 3 September 2015 (2015-09-03)<br>* paragraphs [0015], [0044], [0107], [0197]; figures 27,28 * | 1-6, 9-11,15 | |
| X<br><br><br><br><br><br>A | MICHAEL JAEGER ET AL: "Reduction of background in optoacoustic image sequences obtained under tissue deformation", JOURNAL OF BIOMEDICAL OPTICS, vol. 14, no. 5, 1 January 2009 (2009-01-01), page 054011, XP055087210, ISSN: 1083-3668, DOI: 10.1117/1.3227038<br>* abstract *<br>* pages 054011-2, left-hand column, paragraph 1 *<br>* pages 054011-5, right-hand column, paragraph 1 - pages 054011-8, right-hand column, paragraph 1; figures 6,8 * | 1-7<br><br><br><br><br><br>12,13 | TECHNICAL FIELDS SEARCHED (IPC)<br>A61B<br>G06T |
| X | WO 2014/016600 A1 (BAMBER J C) 30 January 2014 (2014-01-30)<br>* abstract; figures 1,7 *<br>& US 2015/182122 A1 (BAMBER JEFFREY COLIN [GB] ET AL) 2 July 2015 (2015-07-02)<br>* paragraphs [0045], [0047], [0048], [0054], [0076], [0077], [0083], [0093], [0097], [0110], [0116]; figures 1,7 * | 1-8 | |

-/--

The present search report has been drawn up for all claims

| Place of search | Date of completion of the search | Examiner |
|---|---|---|
| Munich | 10 March 2016 | Daoukou, Eleni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 15 18 6402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/125853 A1 (FUJIFILM CORP [JP]) 21 August 2014 (2014-08-21) * abstract; figures 1,4,5-9 * | 1-6,8,15 | |
| Y | US 2011/232385 A1 (NANAUMI RYUICHI [JP]) 29 September 2011 (2011-09-29) * paragraphs [0009], [0027], [0032], [0045] - [0047], [0051]; figures 4,6 * | 12,13 | |
| A | US 2014/243633 A1 (ADDISON PAUL STANLEY [GB] ET AL) 28 August 2014 (2014-08-28) * paragraphs [0048], [0054], [0056]; figures 1,2a,2b * | 1-3,11, 15 | |
| A | JAEGER M ET AL: "Improved contrast deep optoacoustic imaging using displacement-compensated averaging: breast tumour phantom studies;Improved contrast deep optoacoustic imaging of breast phantoms", PHYSICS IN MEDICINE AND BIOLOGY, INSTITUTE OF PHYSICS PUBLISHING, BRISTOL GB, vol. 56, no. 18, 18 August 2011 (2011-08-18), pages 5889-5901, XP020210558, ISSN: 0031-9155, DOI: 10.1088/0031-9155/56/18/008 * page 5898, paragraph 2; figure 7 * | 9 | |
| | | | TECHNICAL FIELDS SEARCHED (IPC) |

The present search report has been drawn up for all claims

| Place of search | Date of completion of the search | Examiner |
|---|---|---|
| Munich | 10 March 2016 | Daoukou, Eleni |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                                 
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 15 18 6402

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2016

| Patent document cited in search report | | Publication date | Patent family member(s) | | Publication date |
|---|---|---|---|---|---|
| US 2010049049 | A1 | 25-02-2010 | JP | 5460000 B2 | 02-04-2014 |
| | | | JP | 2010046215 A | 04-03-2010 |
| | | | US | 2010049049 A1 | 25-02-2010 |
| | | | US | 2014378814 A1 | 25-12-2014 |
| WO 2014063005 | A1 | 24-04-2014 | US | 2015245771 A1 | 03-09-2015 |
| | | | WO | 2014063005 A1 | 24-04-2014 |
| WO 2014016600 | A1 | 30-01-2014 | EP | 2877083 A1 | 03-06-2015 |
| | | | JP | 2015522385 A | 06-08-2015 |
| | | | US | 2015182122 A1 | 02-07-2015 |
| | | | WO | 2014016600 A1 | 30-01-2014 |
| WO 2014125853 | A1 | 21-08-2014 | JP | 2014155596 A | 28-08-2014 |
| | | | WO | 2014125853 A1 | 21-08-2014 |
| US 2011232385 | A1 | 29-09-2011 | JP | 5441781 B2 | 12-03-2014 |
| | | | JP | 2011200414 A | 13-10-2011 |
| | | | US | 2011232385 A1 | 29-09-2011 |
| | | | US | 2015007659 A1 | 08-01-2015 |
| US 2014243633 | A1 | 28-08-2014 | NONE | | |

EPO FORM P0459

For more details about this annex : see Official Journal of the European Patent Office, No. 12/82

**EP 3 011 897 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013075000 A